# EUROPEAN PATENT APPLICATION

(11) **EP 4 286 409 A1**
(43) Date of publication of application: **06.12.2023**
(21) Application number: 22736641.6
(22) Date of filing: 11.01.2022
(51) Int. Cl.: C07K 16/00, C12N 5/10, A61K 39/395, A61P 31/00, G01N 33/68

(54) **ANTIGEN-BINDING PROTEIN TARGETING STAPHYLOCOCCUS AUREUS ?-HEMOLYSIS AND APPLICATION THEREOF**

(30) Priority: 11.01.2021 CN 202110033695
(71) Applicant: STARMAB BIOLOGICS (SUZHOU) CO., LTD, Suzhou, Jiangsu 215101 (CN); Starshining Biologics (Shanghai) Co., Ltd, Shanghai 201210 (CN)
(72) Inventor: AN, Maomao, Suzhou, Jiangsu 215101 (CN); QIU, Xiran, Suzhou, Jiangsu 215101 (CN); CHEN, Simin, Suzhou, Jiangsu 215101 (CN); GUO, Shiyu, Suzhou, Jiangsu 215101 (CN); LI, Bohua, Suzhou, Jiangsu 215101 (CN)
(74) Representative: Arnold & Siedsma
(86) International application number: PCT/CN2022/071382
(87) International publication number: WO 2022/148480

(57) **Abstract**

The present invention relates to the field of biomedicine, and provides an isolated antigen-binding protein and a derived protein thereof, which can specifically bind to *Staphylococcus aureus* α-hemolysis and neutralize the biological activity thereof. Also provided is a use of the isolated antigen-binding protein and derived protein thereof in the field of treating and/or preventing *Staphylococcus aureus* infection.

## Description

### Technical field

This application relates to the field of biomedicine, in particular to an antigen binding protein targeting *Staphylococcus aureus* α-toxin.

### Background

*Staphylococcus aureus,* which belongs to the genus Staphylococcus, is an important Gram-positive pathogen. It can not only cause community-acquired respiratory system infection, skin and soft tissue infection, but also cause various infections after hospital ventilator-associated pneumonia, surgery and various invasive operations (such as tracheotomy, venous catheterization, etc.). In 2019, the data from national drug resistance monitoring website (http://www.carss.cn/) showed that the clinical detection rate of *Staphylococcus aureus* ranked fourth among all pathogenic bacteria and first among G+ pathogenic bacteria. Due to the continuous development and large-scale application of antibacterial drugs, *Staphylococcus aureus* is constantly mutated, and the phenomenon of drug resistance is increasingly serious.

α-Toxin is the most important exotoxins released by *Staphylococcus aureus,* which plays the most important role in its pathogenic process. Alpha toxin is a secreted toxin protein encoded by *HLA* gene of *Staphylococcus aureus,* which is expressed in almost all strains and is the most critical virulence factor affecting the pathogenicity of *Staphylococcus aureus.* Alpha toxin can bind with cholesterol and sphingomyelin on the host cell membrane and aggregate to form a heptamer, and then fold to form a β-barrel transmembrane structure with a diameter of about 1.5 nm, which can rapidly lyse host red blood cells and other tissue cells. At the same time, alpha toxin can also destroy the white blood cells in the infected tissue and hinder the host from clearing the infected *Staphylococcus aureus.* Under the pressure of the host's immune system as well as antibiotics, *Staphylococcus aureus* at the infection site releases a large amount of alpha toxin, which can activate, after entering the blood, the host's immune system to release excessive inflammatory factors and causing sepsis.

At present, the increasing resistance of *Staphylococcus aureus* to antibiotics has complicated the problem of *Staphylococcus aureus* infection. In the clinical treatment of *Staphylococcus aureus* infection, small molecule anti-infection drugs can not solve the body damage and sepsis caused by toxins. Therefore, it is urgent to develop effective alternative methods for the diagnosis and treatment of *Staphylococcus aureus* infection.

### Summary of invention

The present application provides an isolated antigen binding protein targeting *Staphylococcus aureus* alpha toxin, which has one or more of the following properties: 1) it can specifically bind to *Staphylococcus aureus* alpha toxin; 2) it is able to neutralize biological activity of *Staphylococcus aureus* alpha toxin; 3) it has pharmacodynamic effects on diseases and conditions caused by *Staphylococcus aureus* infection; 4) its biological function is better than the published *Staphylococcus aureus* antibody molecules. The present application also provides nucleic acid molecules encoding the isolated antigen binding protein, expression vectors, host cells, immunoconjugates containing the antigen binding protein, pharmaceutical compositions, methods for preparing the isolated antigen binding protein, and the use of the isolated antigen binding protein described herein.

In one aspect, the present application provides an isolated antigen binding protein, which contains HCDR3 that comprises an amino acid sequence shown in SEQ ID NO: 1, SEQ ID NO: 14 or SEQ ID NO: 26.

In some embodiments, the isolated antigen binding protein contains HCDR2 that comprises an amino acid sequence shown in SEQ ID NO: 52 or SEQ ID NO: 27.

In some embodiments, the HCDR2 comprises an amino acid sequence shown in SEQ ID NO: 2 or SEQ ID NO: 15.

In some embodiments, the isolated antigen binding protein contains HCDR1 that comprises an amino acid sequence shown in SEQ ID NO: 53 or SEQ ID NO: 28.

In some embodiments, the HCDR1 comprises an amino acid sequence shown in SEQ ID NO: 3 or SEQ ID NO: 16.

In some embodiments, the isolated antigen binding protein comprises HCDR1, HCDR2 and HCDR3, the HCDR1 comprises an amino acid sequence shown in SEQ ID NO: 53 or SEQ ID NO: 28, the HCDR2 comprises an amino acid sequence shown in SEQ ID NO: 52 or SEQ ID NO: 27, and the HCDR3 comprises an amino acid sequence shown in SEQ ID NO: 1, SEQ ID NO: 14 or SEQ ID NO: 26.

In some embodiments, the isolated antigen binding protein comprises HCDR1, HCDR2 and HCDR3, the HCDR1 comprises an amino acid sequence shown in any one of SEQ ID NO: 3, SEQ ID NO: 16 and SEQ ID NO: 28, the HCDR2 comprises an amino acid sequence shown in any one of SEQ ID NO: 2, SEQ ID NO: 15 and SEQ ID NO: 27, and the HCDR3 comprises an amino acid sequence shown in any one of SEQ ID NO: 1, SEQ ID NO: 14 and SEQ ID NO: 26.

In some embodiments, the isolated antigen binding protein comprises HCDR1, HCDR2, and HCDR3 selected from any of the following groups:
1) The HCDR1 contains an amino acid sequence shown in SEQ ID NO: 3, the HCDR2 contains an amino acid sequence shown in SEQ ID NO: 2, and the HCDR3 contains an amino acid sequence shown in SEQ ID NO: 1;
2) The HCDR1 contains an amino acid sequence shown in SEQ ID NO: 16, the HCDR2 contains an amino acid sequence shown in SEQ ID NO: 15, and the HCDR3 contains an amino acid sequence shown in SEQ ID NO: 14; and
3) The HCDR1 contains an amino acid sequence shown in SEQ ID NO: 28, the HCDR2 contains an amino acid sequence shown in SEQ ID NO: 27, and the HCDR3 contains an amino acid sequence shown in SEQ ID NO: 26.

In some embodiments, the isolated antigen binding protein comprises H-FR1, the C terminus of which is directly or indirectly linked to the N terminus of the HCDR1, and the H-FR1 comprises an amino acid sequence shown in SEQ ID NO: 54.

In some embodiments, the H-FR1 comprises an amino acid sequence shown in any one of SEQ ID NO: 4, SEQ ID NO: 8, SEQ ID NO: 17, SEQ ID NO: 29 and SEQ ID NO: 33.

In some embodiments, the isolated antigen binding protein comprises H-FR2, the H-FR2 is located between the HCDR1 and HCDR2, and the H-FR2 comprises an amino acid sequence shown in SEQ ID NO: 55 or SEQ ID NO: 56.

In some embodiments, the H-FR2 comprises an amino acid sequence shown in any one of SEQ ID NO: 5, SEQ ID NO: 9, SEQ ID NO: 18, SEQ ID NO: 21, SEQ ID NO: 30 and SEQ ID NO: 34.

In some embodiments, the isolated antigen binding protein comprises H-FR3, the H-FR3 is located between the HCDR2 and the HCDR3, and the H-FR3 comprises an amino acid sequence shown in SEQ ID NO: 57 or SEQ ID NO: 58.

In some embodiments, the H-FR3 comprises an amino acid sequence shown in any one of SEQ ID NO: 6, SEQ ID NO: 10, SEQ ID NO: 19, SEQ ID NO: 22, SEQ ID NO: 31 and SEQ ID NO: 35.

In some embodiments, the isolated antigen binding protein comprises H-FR4, the N terminus of which is linked to the C terminus of the HCDR3, and the H-FR4 comprises an amino acid sequence shown in SEQ ID NO: 59.

In some embodiments, the H-FR4 comprises an amino acid sequence shown in any one of SEQ ID NO: 7, SEQ ID NO: 11, SEQ ID NO: 20, SEQ ID NO: 23, SEQ ID NO: 32 and SEQ ID NO: 36.

In some embodiments, the isolated antigen binding protein comprises H-FR1, H-FR2, H-FR3 and H-FR4, the H-FR1 comprises an amino acid sequence shown in SEQ ID NO: 54, the H-FR2 comprises an amino acid sequence shown in SEQ ID NO: 55 or SEQ ID NO: 56, the H-FR3 comprises an amino acid sequence shown in SEQ ID NO: 57 or SEQ ID NO: 58, and the H-FR4 comprises an amino acid sequence shown in SEQ ID NO: 59.

In some embodiments, the isolated antigen binding protein comprises H-FR1, H-FR2, H-FR3 and H-FR4, the H-FR1 comprises an amino acid sequence shown in any one of SEQ ID NO: 4, SEQ ID NO: 8, SEQ ID NO: 17, SEQ ID NO: 29 and SEQ ID NO: 33, the H-FR2 comprises an amino acid sequence shown in any one of SEQ ID NO: 5, SEQ ID NO: 9, SEQ ID NO: 18, SEQ ID NO: 21, SEQ ID NO: 30 and SEQ ID NO: 34, the H-FR3 comprises an amino acid sequence shown in any one of SEQ ID NO: 6, SEQ ID NO: 10, SEQ ID NO: 19, SEQ ID NO: 22, SEQ ID NO: 31 and SEQ ID NO: 35, and the H-FR4 comprises an amino acid sequence shown in any one of SEQ ID NO: 7, SEQ ID NO: 11, SEQ ID NO: 20, SEQ ID NO: 23, SEQ ID NO: 32 and SEQ ID NO: 36.

In some embodiments, the isolated antigen binding protein comprises H-FR1, H-FR2, H-FR3, and H-FR4 selected from any of the following groups:
1) The H-FR1 contains an amino acid sequence shown in SEQ ID NO: 4, the H-FR2 contains an amino acid sequence shown in SEQ ID NO: 5, the H-FR3 contains an amino acid sequence shown in SEQ ID NO: 6, and the H-FR4 contains an amino acid sequence shown in SEQ ID NO: 7;
2) The H-FR1 contains an amino acid sequence shown in SEQ ID NO: 8, the H-FR2 contains an amino acid sequence shown in SEQ ID NO: 9, the H-FR3 contains an amino acid sequence shown in SEQ ID NO: 10, and the H-FR4 contains an amino acid sequence shown in SEQ ID NO: 11;
3) The H-FR1 contains an amino acid sequence shown in SEQ ID NO: 17, the H-FR2 contains an amino acid sequence shown in SEQ ID NO: 18, the H-FR3 contains an amino acid sequence shown in SEQ ID NO: 19, and the H-FR4 contains an amino acid sequence shown in SEQ ID NO: 20;
4) The H-FR1 contains an amino acid sequence shown in SEQ ID NO: 8, the H-FR2 contains an amino acid sequence shown in SEQ ID NO: 21, the H-FR3 contains an amino acid sequence shown in SEQ ID NO: 22, and the H-FR4 contains an amino acid sequence shown in SEQ ID NO: 23;
5) The H-FR1 contains an amino acid sequence shown in SEQ ID NO: 29, the H-FR2 contains an amino acid sequence shown in SEQ ID NO: 30, the H-FR3 contains an amino acid sequence shown in SEQ ID NO: 31, and the H-FR4 contains an amino acid sequence shown in SEQ ID NO: 32; and
6) The H-FR1 contains an amino acid sequence shown in SEQ ID NO: 33, the H-FR2 contains an amino acid sequence shown in SEQ ID NO: 30, the H-FR3 contains an amino acid sequence shown in SEQ ID NO: 35, and the H-FR4 contains an amino acid sequence shown in SEQ ID NO: 36.

In some embodiments, the isolated antigen binding protein comprises VH, which comprises an amino acid sequence shown in SEQ ID NO: 60 or SEQ ID NO: 61.

In some embodiments, the VH comprises an amino acid sequence shown in any one of SEQ ID NO: 12, SEQ ID NO: 13, SEQ ID NO: 24, SEQ ID NO: 25, SEQ ID NO: 37 and SEQ ID NO: 38.

In some embodiments, the isolated antigen binding protein comprises VHH.

In some embodiments, the VHH comprises an amino acid sequence shown in SEQ ID NO: 60 or SEQ ID NO: 61.

In some embodiments, the VHH comprises an amino acid sequence shown in any one of SEQ ID NO: 12, SEQ ID NO: 13, SEQ ID NO: 24, SEQ ID NO: 25, SEQ ID NO: 37 and SEQ ID NO: 38.

In some embodiments, the isolated antigen binding protein comprises a heavy chain constant region of an antibody.

In some embodiments, the heavy chain constant region of an antibody is derived from the human IgG constant region.

In some embodiments, the heavy chain constant region of an antibody is derived from the human IgG1 constant region.

In some embodiments, the heavy chain constant region of an antibody comprises an amino acid sequence as shown in SEQ ID NO: 39.

In some embodiments, the isolated antigen binding protein comprises an amino acid sequence shown in any one of SEQ ID NO: 62, SEQ ID NO: 63, SEQ ID NO: 64, SEQ ID NO: 68, SEQ ID NO: 69 and SEQ ID NO: 70.

In some embodiments, the isolated antigen binding protein includes an antibody or an antigen binding fragment thereof.

In some embodiments, the antigen binding fragment includes Fab, Fab', Fv fragment, Bs-Fv, F(ab')₂, F(ab)₂, scFv, di-scFv and/or dAb.

In some embodiments, the antibody is selected from one or more of the following groups: monoclonal antibody, chimeric antibody, humanized antibody, and fully human antibody.

In some embodiments, the isolated antigen binding protein can specifically bind to *Staphylococcus aureus* alpha toxin.

In some embodiments, the isolated antigen binding protein can neutralize the biological activity of the *Staphylococcus aureus* alpha toxin.

In some embodiments, the isolated antigen binding protein can prevent and/or treat diseases and/or conditions and their complications.

In some embodiments, the disease and/or condition and complication thereof is caused or mediated by *Staphylococcus aureus.*

In some embodiments, the disease and/or condition and complication thereof includes bacteremia and/or sepsis.

In another aspect, the present application also provides a polypeptide molecule comprising the isolated antigen binding protein.

In some embodiments, the polypeptide molecule comprises a fusion protein.

In some embodiments, the polypeptide molecule comprises two or more isolated antigen binding proteins or a fusion protein of the two or more isolated antigen binding proteins and an antibody heavy chain constant region.

In some embodiments, the polypeptide molecule comprises an amino acid sequence shown in any one of SEQ ID NO: 65, SEQ ID NO: 66 and SEQ ID NO: 67.

In another aspect, the present application also provides an immunoconjugate comprising the isolated antigen binding protein.

In another aspect, the present application also provides a nucleic acid molecule, which encodes the isolated antigen binding protein or the polypeptide molecule.

In another aspect, the present application also provides a vector comprising the nucleic acid molecule.

In another aspect, the present application also provides a cell comprising the nucleic acid molecule or the vector.

In another aspect, the present application also provides a pharmaceutical composition comprising the isolated antigen binding protein, the polypeptide molecule, the immunoconjugate, the nucleic acid molecule, the vector and/or the cell, and optionally a pharmaceutically acceptable carrier.

In another aspect, the present application also provides a method for preparing the isolated antigen binding protein, which comprises culturing the cell under the condition that the antigen binding protein is expressed.

In another aspect, the present application also provides a use of the isolated antigen binding protein, the polypeptide molecule, the immunoconjugate, the nucleic acid molecule, the vector, the cell and/or the pharmaceutical composition in the manufacture of a medicament for the prevention and/or treatment of diseases and/or disorders and their complications.

In some embodiments, the disease and/or condition and complication thereof is caused or mediated by *Staphylococcus aureus.*

In some embodiments, the disease and/or condition and complication thereof includes sepsis and/or bacteremia.

In another aspect, the isolated antigen binding protein, the polypeptide molecule, the immunoconjugate, the nucleic acid molecule, the vector, the cell and/or the pharmaceutical composition described herein are used alone or in combination with other molecules.

In another aspect, the present application also provides a method for detecting *Staphylococcus aureus* alpha toxin in a sample, wherein the method comprises using the isolated antigen binding protein, the polypeptide molecule, the immunoconjugate, the nucleic acid molecule, the vector, the cell and/or the pharmaceutical composition.

In another aspect, the present application also provides a reagent or kit for detecting *Staphylococcus aureus* alpha toxin in a sample, comprising the isolated antigen binding protein, the polypeptide molecule, the immunoconjugate, the nucleic acid molecule, the vector, the cell, and/or the pharmaceutical composition.

In another aspect, the present application also provides use of the isolated antigen binding protein, the polypeptide molecule, the immunoconjugate, the nucleic acid molecule, the vector, the cell and/or the pharmaceutical composition in the manufacture of a kit for detecting the presence and/or content of *Staphylococcus aureus* alpha toxin in a sample.

Those skilled in the art can easily perceive other aspects and advantages of the present application from the following detailed description. Only exemplary embodiments of the present application are shown and described in the following detailed description. As those skilled in the art will recognize, the content of the present application enables those skilled in the art to make changes to the disclosed specific embodiments without departing from the spirit and scope of the invention involved in the present application. Accordingly, the descriptions in the drawings and specification of the present application are merely exemplary and not restrictive.

### Description of Drawings

The specific features of the invention involved in the present application are shown in the appended claims. The features and advantages of the invention according to the present application can be better understood by referring to the exemplary embodiments and figures described in detail below. A brief description of the attached figures is as follows:
Fig. 1 shows the ELISA detection of the binding ability of the antigen binding protein described herein to *Staphylococcus aureus* alpha toxin protein.
Fig. 2 shows the neutralization effect of the antigen binding protein described herein on the hemolytic activity of recombinant *Staphylococcus aureus* alpha toxin.
Fig. 3 shows the therapeutic effect of the antigen binding protein described herein on sepsis induced by *Staphylococcus aureus* alpha toxin in mice.
Fig. 4 shows the therapeutic effect of the antigen binding protein described herein on bacteremia caused by *Staphylococcus aureus* in mice.
Fig. 5 shows the ELISA detection of the binding ability of the antigen binding protein described herein to *Staphylococcus aureus* alpha toxin protein.
Fig. 6 shows the neutralization effect of the antigen binding protein described herein on the hemolytic activity of recombinant *Staphylococcus aureus* alpha toxin.
Fig. 7 shows the therapeutic effect of the antigen binding protein described herein on sepsis induced by *Staphylococcus aureus* alpha toxin in mice.
Fig. 8 shows the therapeutic effect of the antigen binding protein described herein on bacteremia caused by *Staphylococcus aureus* in mice.
Fig. 9 shows the neutralization effect of the quadrivalent antigen binding protein described herein on the hemolytic activity of *Staphylococcus aureus* alpha toxin.
Fig. 10 shows the neutralization effect of the quadrivalent antigen binding protein described herein on the hemolytic activity of *Staphylococcus aureus* alpha toxin.
Fig. 11 shows the neutralization effect of the tetravalent antigen binding protein described herein on the hemolytic activity of *Staphylococcus aureus* alpha toxin.

### Embodiments

The following is a description of the embodiment of the invention according to the present application by specific examples. Those skilled in the art can easily understand other advantages and effects of the invention according to the contents disclosed in the specification.

### Definition

In the present application, the term *"Staphylococcus aureus"* can be used interchangeably with *"S. aureus", "Staph. aureus",* which usually belongs to gram positive bacteria. In the present application, the term can cover any kind and form of *Staphylococcus aureus* and its variants, homologs and functionally active fragments.

In the present application, the term *"Staphylococcus aureus* alpha toxin" is also called "α-toxin", "α-hemolysin", "hemolysin A", "Hla", "hemolysin A toxin", which usually refers to a pore forming protein hemolysin produced by *Staphylococcus aureus.* In the present application, the *Staphylococcus aureus* alpha toxin can include wild-type *Staphylococcus aureus* alpha toxin and its variants, derivatives, homologs, recombinant proteins, monomers, oligomers and other forms. The oligomeric form may include different forms of combination of alpha toxin monomers, such as dimer, trimer, tetramer, pentamer, hexamer, heptamer, or multimeric forms, such as heptamer pre pore form of alpha toxin.

In the present application, the term "isolated" usually refers to those obtained by artificial means from a natural state. If an "isolated" substance or component appears in nature, its natural environment may have been changed, or the substance may be separated from the natural environment, or both. For example, a living animal naturally has a certain kind of undissociated polynucleotide or polypeptide, and the same polynucleotide or polypeptide with high purity isolated from this natural state is called isolated. The term "isolated" does not exclude the mixing of artificial or synthetic substances, nor does it exclude the presence of other impure substances that do not affect the activity of the substance.

In the present application, the term "isolated antigen binding protein" generally refers to a protein with antigen binding ability that is separated from its naturally occurring state. The "isolated antigen binding protein" may comprise a portion that binds antigen, and optionally a framework or framework portion that allows the antigen binding portion to adopt the conformation that facilitates the antigen binding portion to bind to the antigen. The antigen binding protein may comprise, for example, antibody derived protein framework regions (FRS) or alternative protein framework regions or artificial framework regions with transplanted CDRs or CDR derivatives. Such frameworks include, but are not limited to, antibody derived framework regions containing mutations introduced, for example, to stabilize the three-dimensional structure of the antigen binding protein and fully synthesized framework regions containing, for example, biocompatible polymers. See, for example, Korndorfer et. al., 2003, Proteins: Structure, Function, and Bioinformatics, 53(1):121-129(2003); Roque et. al., Biotechnol. Prog. 20:639-654 (2004). Examples of the antigen binding protein include, but are not limited to: human antibody; humanized antibody; chimeric antibody; recombinant antibody; single chain antibody; bifunctional antibody; trifunctional antibody; tetrafunctional antibody; Fab, Fab', Fv fragment, Bs-Fv, F(ab')₂, F(ab)₂, scFv, di-scFv, dAb, IgD antibody; IgE antibody; IgM antibody; IgG1 antibody; IgG2 antibody; IgG3 antibody; or IgG4 antibody and fragments thereof.

In the present application, the term "CDR", also known as "complementarity determining region", usually refers to a region in the variable domain of an antibody whose sequence is highly variable and/or forms a structure defining loop. Typically, antibodies include six CDRs; three in VH (HCDR1, HCDR2, HCDR3), and three in VL (LCDR1, LCDR2, LCDR3). In some embodiments, naturally occurring camel antibodies composed of only heavy chains can also function normally and stably in the absence of light chains. See, for example, Hamers-Casterman et. al., Nature 363:446-448 (1993); Sheriff et. al., Nature Struct. Biol. 3:733-736 (1996). Antibody CDRs can be determined by a variety of coding systems, such as CCG, Kabat, AbM, Chothia, IMGT, combined Kabat/Chothia, etc. These coding systems are known in the art. For details, see, for example, http://www.bioinf.org.uk/abs/index.html#kabatnum. For example, the amino acid sequence of the antigen binding protein can be numbered according to the IMGT (the international ImMunoGeneTics information system@imgt.cines.fr; http: //imgt.cines.fr; http://imgt.cines.fr; Lefranc et. al., 1999, Nucleic Acids Res. 27: 209-212; Ruiz et. al.,2000 Nucleic Acids Res. 28: 219-221; Lefranc et. al., 2001, Nucleic Acids Res. 29:207-209; Lefranc et. al., 2003, Nucleic Acids Res. 31: 307-310; Lefranc et. al., 2005, DevComp Immunol 29: 185-203). For example, the CDRs of the antigen binding protein can be determined according to the Kabat numbering system (see, for example, Kabat EA &Wu TT (1971) Ann NY AcadSci 190:382-391 and Kabat EA et. al., (1991) Sequences of Proteins of Immunological Interest, FifthEdition, U.S. Department of Health and Human Services, NIH Publication No.91-3242).

In the present application, the term "FR" generally refers to a more highly conserved part of the antibody variable domain, which is referred to as the framework region. In general, the variable domains of natural heavy and light chains each contain four FR regions, namely four in VH (H-FR1, H-FR2, H-FR3, and H-FR4), and four in VL (L-FR1, L-FR2, L-FR3, and L-FR4).

In the present application, the terms "variable domain" and "variable region" can be used interchangeably, usually referring to a part of the antibody heavy chain and/or light chain. The variable domains of the heavy and light chains can be referred to as "V_{H}" and "V_{L}" (or "VH" and "VL", respectively). These domains are usually the most variable parts of antibodies (relative to other antibodies of the same type) and contain antigen binding sites.

In the present application, the term "variable" generally refers to that there may be large differences in the sequence of some segments of the variable domain between antibodies. Variable domains mediate antigen binding and determine the specificity of a specific antibody to its specific antigen. However, the variability is not evenly distributed throughout the variable domain. It is usually concentrated in three segments called hypervariable regions (CDRs or HVRs) in the light and heavy chain variable domains. The more highly conserved part of the variable domain is called the framework region (FR). The variable domains of natural heavy and light chains each contain four FR regions, most of which are in β-sheet configuration, connected by three CDRs, which form a ring linkage, and in some cases a part of a β-sheet structure. CDRs in each chain are held together closely by the FR regions, and CDRs from the other chain together promote the formation of antigen binding sites of antibodies (see Kabat et. al., Sequences of Immunological Interest, Fifth Edition, National Institute of Health, Bethesda, Md. (1991)).

In the present application, the term "antibody" generally refers to immunoglobulin or fragments thereof or derivatives thereof, covering any polypeptide including an antigen binding site, no matter whether it is produced *in vitro* or *in vivo.* The term includes but is not limited to polyclonal, monoclonal, monospecific, multispecific, nonspecific, humanized, single chain, chimeric, synthetic, recombinant, hybrid, mutant and transplanted antibodies. Unless otherwise modified by the term "complete", such as in "complete antibody", for the purpose of the present invention, the term "antibody" also includes antibody fragments, such as Fab, F(ab')₂, Fv, scFv, Fd, dAb, and other antibody fragments that maintain antigen binding function (for example, specifically bind to *Staphylococcus aureus* alpha toxin). Generally, such fragments should include an antigen binding domain.

The basic 4-chain antibody unit is a heterotetrameric glycoprotein composed of two identical light chains (L) and two identical heavy chains (H). IgM antibody consists of 5 basic heterotetramer units and another polypeptide called J chain, which contains 10 antigen binding sites; IgA antibody contains 2-5 basic 4 chain units, which can polymerize with J chain to form a multivalent assemblages. In the case of IgGs, the 4-chain unit is typically about 150,000 daltons. Each light chain is connected to a heavy chain through a covalent disulfide bond, while the two heavy chains are connected to each other through one or more disulfide bonds, and the number of disulfide bonds depends on the isotype of the heavy chain. Each heavy and light chain also has an interchain disulfide bridge with regular spacing. Each heavy chain has a variable domain (VH) at the N-terminus, followed by three (for each α and γ chain) and four (for µ and ε isoforms) constant domains (CH). Each light chain has a variable domain (VL) at the N-terminus, followed by a constant domain (CL) at the other end. VL is aligned with VH, while CL is aligned with the first constant domain (CH1) of the heavy chain. Specific amino acid residues are thought to form an interface between light and heavy chain variable domains. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6. The L chain from any vertebrate species can be assigned to one of two clearly distinct types, called kappa and lambda, based on the amino acid sequences of their constant domains.. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α, δ, ε, γ and µ, respectively.

In the present application, the term "antigen binding fragment" generally refers to one or more fragments with the ability to specific bind to antigen (e.g., *Staphylococcus aureus* alpha toxin). In the present application, the antigen binding fragment may include Fab, Fab', F(ab)₂, Fv fragment, F(ab')₂, scFv, di-scFv and/or dAb.

In the present application, the term "Fab" generally refers to the antigen binding fragment of an antibody. As mentioned above, papain can be used to digest intact antibodies. The antibody digested by papain produces two identical antigen binding fragments, namely, "Fab" fragment and residual "Fc" fragment (namely Fc region, *ibid.).* The Fab fragment may consist of a complete L chain, a variable region of a heavy chain, and the first constant region (CH1) of the H chain (VH).

In the present application, the term "Fab' fragment" usually refers to the monovalent antigen binding fragment of a human monoclonal antibody, which is slightly larger than the Fab fragment. For example, Fab' fragments may include all light chains, all heavy chain variable regions, and all or part of the first and second constant regions of the heavy chain. For example, the Fab' fragment may also include some or all of the 220-330 amino acid residues of the heavy chain.

In the present application, the term "F(ab')2" generally refers to the antibody fragment generated by pepsin digestion of the complete antibody. The F(ab')2 fragment contains two Fab fragments and part of the hinge region maintained together by disulfide bonds. The F(ab')2 fragment has bivalent antigen binding activity and can cross link antigens.

In the present application, the term "Fv fragment" generally refers to the monovalent antigen binding fragment of a human monoclonal antibody, including all or part of the heavy chain variable region and light chain variable region, and lacks the heavy chain constant region and light chain constant region. The heavy chain variable region and the light chain variable region include, for example, CDRs. For example, Fv fragments include all or part of the amino terminal variable regions of about 110 amino acids of the heavy and light chains.

In the present application, the term "scFv" generally refers to a fusion protein comprising at least one antibody fragment including the variable region of the light chain and at least one antibody fragment including the variable region of the heavy chain, wherein the variable region of the light chain and the heavy chain are adjacent (for example, via a synthetic linker such as a short flexible polypeptide linker), and can be expressed as a single chain polypeptide, and wherein the scFv retains the specificity of the intact antibody from which it is derived. Unless otherwise specified, as used in the present application, scFv can have the VL and VH variable regions in any order (such as relative to the N-terminus and C-terminus of the polypeptide), and scFv can include VL-linker-VH or VH-linker-VL.

In the present application, the term "dAb" generally refers to an antigen binding fragment with a VH domain, a VL domain, or a VH domain or a VL domain, with reference to, for example, Ward et. al. (Nature, 1989oct 12; 341 (6242):544-6), Holt et. al., Trends Biotechnol, 2003,21 (11):484-490; and referring to WO 06/030220, WO 06/003388 and other published patent applications by DomantisLtd. The term "dAb" generally includes sdAb. The term "sdAb" usually refers to a single domain antibody. Single domain antibodies usually refer to antibody fragments that consist only of the variable region of the antibody heavy chain (VH domain) or the variable region of the antibody light chain (VL).

In the present application, the term "VHH" usually refers to the variable antigen binding domain of heavy chain antibodies from camelidae (camels, dromedaries, llamas, alpacas, etc.) (see Nguyen V.K. et. al., 2000, The EMBO Journal, 19, 921-930; Muyldermans S., 2001, J Biotechnol., 74, 277-302 and a review of Vanlandschoot P. et. al., 2011, Antiviral Research 92, 389-407). VHH can also be called Nanobody (Nb).

In the present application, the term "monoclonal antibody" generally refers to an antibody molecule preparation consisting of a single molecule. Monoclonal antibodies are usually highly specific for a single antigen site. Monoclonal generally antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made in hybridoma cells or by recombinant DNA method.

In the present application, the term "chimeric antibody" generally refers to an antibody in which the variable region is derived from one species and the constant region is derived from another species. In general, the variable region is derived from antibodies of experimental animals such as rodents ("parental antibodies"), and the constant region is derived from human antibodies, causing the resulting chimeric antibodies less likely to elicit adverse immune reactions in human individuals than parental (e.g., alpaca derived) antibodies.

In the present application, the term "humanized antibody" generally refers to an antibody whose part or all of the amino acids outside the CDR region of a non-human antibody (such as an alpaca antibody) are replaced by the corresponding amino acids derived from human immunoglobulin. In the CDR region, small additions, deletions, insertions, substitutions or modifications of amino acids can also be allowed, as long as they still retain the ability of antibodies to bind specific antigens. The humanized antibody may optionally comprise at least a portion of the constant region of human immunoglobulin. "Humanized" forms of non-human (e.g., alpaca) antibodies are chimeric antibodies that contain minimal sequence derived from non-human immunoglobulin. In one embodiment, a humanized antibody is a human immunoglobulin (recipient antibody) in which residues from an HVR (hereinafter defined) of the recipient are replaced by residues from an HVR of a non-human species (donor antibody) such as alpaca, mouse, rat, rabbit or non-human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity.

The term "fully human antibody" generally refers to an antibody that contains only the human immunoglobulin protein sequence. If it is produced in mice, in mouse cells, or in hybridomas derived from mouse cells, the fully human antibody may contain murine glycans. Similarly, "mouse antibody" or "rat antibody" refer to antibodies containing only mouse or rat immunoglobulin sequences, respectively. Fully human antibody can be generated in human body and transgenic animals with human immunoglobulin germline sequence by phage display or other molecular biological methods. Exemplary techniques that can be used in the manufacture of antibodies are described in US patents: 6,150,584, 6,458,592, 6,420,140. Other techniques, such as the use of libraries, are known in the art.

In the present application, the terms "polypeptide molecule", "polypeptide" and "peptide" can be used interchangeably, usually referring to polymers of amino acid residues. The term "fusion protein" generally refers to a polypeptide with at least two covalently linked moieties. Each part can be a polypeptide with different properties. This property can be a biological property, such as *in vitro* or *in vivo* activity. This property can also be a simple chemical or physical property, such as binding to target molecules, catalysis of reactions, etc. These two parts can be directly connected through a single peptide bond or through a peptide linker.

In the present application, the term "nucleic acid molecule" generally refers to a nucleotide, deoxyribonucleotide or ribonucleotide in isolated form of any length, or an analog isolated from its natural environment or synthesized artificially.

In the present application, the term "vector" generally refers to a nucleic acid delivery vehicle that can insert a polynucleotide encoding a protein into it and enable the protein to be expressed. The vector can be transformed, transduced or transfected into host cells, so that the genetic material elements carried by it can be expressed in host cells. For example, vectors may include: plasmids; phagemid; cosmid; artificial chromosomes such as yeast artificial chromosome (YAC), bacterial artificial chromosome (BAC) or P1 derived artificial chromosome (PAC); phages such as λ Phage or M13 Phage, and animal virus, etc. Animal viruses used as vectors can include retrovirus (including lentivirus), adenovirus, adeno-associated virus, herpesvirus (such as herpes simplex virus), poxvirus, baculovirus, papillomavirus, papillomavacuolating virus (such as SV40). A vector may contain a variety of elements for controlling expression, including promoter sequence, transcription initiation sequence, enhancer sequence, selection element and reporter gene. In addition, the vector may also contain a replication start site. The vector may also include components that help it enter the cell, such as but not limited to viral particles, liposomes or protein coats.

In the present application, the term "cell" generally refers to a single cell, cell line or cell culture that can be or has been the recipient of the subject plasmid or vector, which includes the nucleic acid molecule or the vector described herein. Cells can include the progeny of a single cell. Due to natural, accidental or intentional mutations, the progeny may not necessarily be identical to the original parent cell (in the morphology of total DNA complement or in the genome). Cells may include cells transfected in vitro with the vector described herein. Cells can be bacterial cells (e.g., *Escherichia coli),* yeast cells, or other eukaryotic cells, such as COS cells, Chinese hamster ovary (CHO) cells, CHO-K1 cells, LNCAP cells, HeLa cells, HEK293 cells, COS-1 cells, NS0 cells.

In the present application, the term "immunoconjugate" generally refers to the conjugate formed by the conjugation of the other reagents (e.g., chemotherapeutic agents, radioactive elements, cell growth inhibitors, and cytotoxic agents) with the antibody or its antigen binding fragment (e.g., covalently linked by a linker molecule), which can specifically bind to the antigen on the target cell through the antibody or its antigen binding fragment to deliver the other reagents to target cells (e.g., tumor cells).

In the present application, the term "pharmaceutical composition" typically refers to a composition used for the prevention/treatment of diseases or conditions. The pharmaceutical composition may include isolated antigen binding proteins, nucleic acid molecules, vectors, and/or cells as described herein, and any pharmaceutically acceptable adjuvants. In addition, the pharmaceutical composition may also include one or more (pharmaceutically effective) vehicles, stabilizers, excipients, diluents, solubilizers, surfactants, emulsifiers, and/or preservatives. The acceptable components of the composition are preferably non-toxic to the recipient at the dosage and concentration used. The pharmaceutical compositions of the present invention include but are not limited to liquid, frozen, and lyophilized compositions.

In the present application, the term "pharmaceutically acceptable vehicle" usually includes pharmaceutically acceptable carriers, excipients or stabilizers, which are non-toxic to cells or mammals exposed to them at the dose and concentration used. Physiologically acceptable carriers may include, for example, buffering agents, antioxidants, low molecular weight (less than about 10 residues) polypeptides, proteins, hydrophilic polymers, amino acids, monosaccharides, disaccharides, and other carbohydrates, chelating agents, sugar alcohols, salt forming counter ions, such as sodium, and/or non-ionic surfactants.

In the present application, the term "specific binding" or "specific" typically refer to measurable and reproducible interactions, such as the binding between a target and an antibody, which can determine the presence of a target in a heterogeneous population of molecules (including biomolecules). For example, antibodies that specifically bind to a target (which can be an epitope) can be antibodies that bind to that target with greater affinity, avidity, ease, and/or longer duration than other targets. In some embodiments, antibodies specifically bind to epitopes on proteins, which are conserved in proteins of different species. In certain embodiments, specific binding may include but not require exclusive binding.

In the present application, the term "subject" typically refers to human or non-human animals, including but not limited to cats, dogs, horses, pigs, cows, sheep, rabbits, mice, rats, or monkeys.

In the present application, the protein, peptide, and/or amino acid sequences involved should also be understood to include at least the following scope: variants or homologs that have the same or similar functions as the protein or peptide.

In the present application, the variant can be, for example, the protein and/or the polypeptide (for example, an antibody that specifically binds to *Staphylococcus aureus* alpha toxin or ots fragments) that has undergone substitution, deletion, or addition of one or more amino acids in the amino acid sequence of the protein or polypeptide. For example, the functional variant may include a protein or polypeptide that has undergone amino acid changes through at least one, such as 1-30, 1-20, or 1-10, and also one, two, three, four, or five amino acid substitutions, deletions, and/or insertions. The functional variant can essentially maintain the biological characteristics of the protein or peptide prior to modification (such as substitution, deletion, or addition). For example, the functional variant can maintain at least 60%, 70%, 80%, 90%, or 100% of the biological activity (such as antigen binding ability) of the protein or polypeptide before the change. For example, the substitution can be conservative.

In the present application, the homolog can be a protein and/or polypeptide that has at least about 85% (for example, at least about 85%, about 90%, about 91%, about 92%, about 93%, about 94%, about 95%, about 96%, about 97%, about 98%, about 99% or more) sequence homology with the protein and/or polypeptide (e.g., an antibody that specifically binds to *Staphylococcus aureus* alpha toxin or fragments thereof).

In the present application, the homology usually refers to the similarity, or association between two or more sequences. The "sequence homology percentage" can be calculated by comparing the two sequences to be compared in the comparison window, determining the number of positions with the same nucleic acid base (such as A, T, C, G, I) or amino acid residue (such as Ala, Pro, Ser, Thr, Gly, Val, Leu, Ile, Phe, Tyr, Trp, Lys, Arg, His, Asp, Glu, Asn, Gln, Cys, and Met) in the wo sequences to obtain the number of matching positions, dividing the number of matching positions by the total number of positions in the comparison window (i.e. window size), and multiplying the result by 100, to produce the sequence homology percentage. The comparison to determine the sequence homology percentage can be conducted in many ways known in the art, for example, using a publicly available computer software such as BLAST, BLAST-2, ALIGN or Megalign (DNASTAR) software. Those skilled in the art can determine suitable parameters for sequence alignment, including any algorithm required to achieve maximum alignment within the full-length sequence range being compared or within the target sequence region. The homology can also be determined by the following methods: FASTA and BLAST. For the description of FASTA algorithm, see W.R. Pearson and D.J. Lipman, "Improved Tool for Biological Sequence Comparison", Proc. Satl. Acad. Sci., 85:2444-2448, 1988; and D.J. Lipman and W.R. Pearson, "Rapid and Sensitive Protein Similarity Search", Science, 227:1435-1441,1989. For the description of BLAST algorithm, see S. Altschul, W. Gish, W. Miller, E.W. Myers, and D. Lipman, "A Basic Local Alignment Search Tool", Journal of Molecular Biology, 215:403-410, 1990.

In the present application, the term "comprise" usually refers to the meaning of including, containing, having, or comprising. In some cases, it also means "being" or "consisting of'.

In the present application, the term "about" usually refers to changes within a range of 0.5% to 10% above or below the specified value, such as changes within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### Detailed description

### Isolated Antigen Binding Proteins

The CDRs of an antibody, also known as the complementarity determining regions, are part of the variable region. The amino acid residues in this region can contact antigen or epitope. The CDRs of an antibody can be determined through various coding systems, such as CCG, Kabat, Chothia, IMGT, AbM, and combined Kabat/Chothia. These coding systems are known in the art and can be found in, for example, http://www.bioinf.org.uk/abs/index.html#kabatnum. Those skilled in the art can determine the CDR region using different coding systems based on the sequence and structure of the antibody. For different coding systems, there may be differences in the CDR regions. In the present application, the CDRs covers CDR sequences obtained by any CDR partitioning method; it also covers their variants, which include the amino acid sequence of the CDR subjected to substitution, deletion, and/or addition with one or more amino acids. For example, 1-30, 1-20, or 1-10, and for example, 1, 2, 3, 4, 5, 6, 7, 8, or 9 amino acid substitutions, deletions, and/or insertions; they also cover their homologues, which can be amino acid sequences that have at least about 85% (for example, have at least about 85%, about 90%, about 91%, about 92%, about 93%, about 94%, about 95%, about 96%, about 97%, about 98%, about 99% or more) sequence homology with the amino acid sequence of the CDRs. In certain embodiments, the isolated antigen binding protein described herein is defined according to the Chothia coding system.

In one aspect, the present application provides an isolated antigen binding protein comprising a HCDR3. In some cases, the HCDR3 may contain an amino acid sequence shown in SEQ ID NO: 1. In some cases, the HCDR3 may contain an amino acid sequence shown in SEQ ID NO: 14. In some cases, the HCDR3 may contain an amino acid sequence shown in SEQ ID NO: 26.

In the present application, the isolated antigen binding protein may also comprise a HCDR2. In some cases, the HCDR2 may contain an amino acid sequence shown in SEQ ID NO: 52.

In some cases, the HCDR2 may contain an amino acid sequence shown in SEQ ID NO: 2. In some cases, the HCDR2 may contain an amino acid sequence shown in SEQ ID NO: 15. In some cases, the HCDR2 may contain an amino acid sequence shown in SEQ ID NO: 28.

In the present application, the isolated antigen binding protein may also comprise a HCDR1. In some cases, the HCDR1 may contain an amino acid sequence shown in SEQ ID NO: 53.

In some cases, the HCDR1 may contain an amino acid sequence shown in SEQ ID NO: 3. In some cases, the HCDR1 may contain an amino acid sequence shown in SEQ ID NO: 16. In some cases, the HCDR1 may contain an amino acid sequence shown in SEQ ID NO: 28.

In the present application, the isolated antigen binding protein may also comprise HCDR1, HCDR2, and HCDR3. For example, the HCDR1 may comprise an amino acid sequence shown in SEQ ID NO: 53 or SEQ ID NO: 28, the HCDR2 may comprise an amino acid sequence shown in SEQ ID NO: 52 or SEQ ID NO: 27, and the HCDR3 can comprise an amino acid sequence shown in SEQ ID NO: 1, SEQ ID NO: 14, or SEQ ID NO: 26.

In the present application, the isolated antigen binding protein comprises HCDR1, HCDR2, and HCDR3, wherein the HCDR1 comprises an amino acid sequence shown in any of SEQ ID NO: 3, SEQ ID NO: 16, and SEQ ID NO: 28, wherein the HCDR2 comprises an amino acid sequence shown in any of SEQ ID NO: 2, SEQ ID NO: 15, and SEQ ID NO: 27, and wherein the HCDR3 comprises an amino acid sequence shown in any of SEQ ID NO: 1, SEQ ID NO: 14, and SEQ ID NO: 26.

For example, the HCDR1 comprises an amino acid sequence shown in SEQ ID NO: 3, the HCDR2 comprises an amino acid sequence shown in SEQ ID NO: 2, and the HCDR3 comprises an amino acid sequence shown in SEQ ID NO: 1.

For example, the HCDR1 comprises an amino acid sequence shown in SEQ ID NO: 16, the HCDR2 comprises an amino acid sequence shown in SEQ ID NO: 15, and the HCDR3 comprises an amino acid sequence shown in SEQ ID NO: 14.

For example, the HCDR1 comprises an amino acid sequence shown in SEQ ID NO: 28, the HCDR2 comprises an amino acid sequence shown in SEQ ID NO: 27, and the HCDR3 comprises an amino acid sequence shown in SEQ ID NO: 26.

In the present application, the isolated antigen binding protein may include at least one CDR from the amino acid sequences shown in SEQ ID NO: 12, SEQ ID NO: 13, SEQ ID NO: 24, SEQ ID NO: 25, SEQ ID NO: 37, and SEQ ID NO: 38. The CDR may include CDRs obtained in any way, but is not limited to CDRs obtained by the partitioning method shown in the table below. If the CDR generated by any method has the same sequence as SEQ ID NO: 12, SEQ ID NO: 13, SEQ ID NO: 24, SEQ ID NO: 25, SEQ ID NO: 37, or SEQ ID NO: 38, it falls within the scope of claims in the present application.

For example, by dividing the amino acid sequences shown in SEQ ID NO: 12 or SEQ ID NO: 13 using the method in Table 1, the isolated antigen binding protein in the present application may comprise CDRs as shown in the following table.

**Table 1. CDR sequences obtained by dividing the amino acid sequences shown in SEQ ID NO: 12 or SEQ ID NO: 13 in different ways**

| **Method** | **CDR1** | **CDR2** | **CDR3** |
|---|---|---|---|
| Chothia | GFTFSRH (SEQ ID NO: 3) | NIPGDD (SEQ ID NO: 2) | AGENMRLVT (SEQ ID NO: 1) |
| AbM | GFTFSRHGMT (SEQ ID NO: 41) | EINIPGDDVD (SEQ ID NO: 40) | AGENMRLVT (SEQ ID NO: 1) |
| Kabat | RHGMT (SEQ ID NO: 43) | EINIPGDDVDYDISVKG (SEQ ID NO: 42) | AGENMRLVT (SEQ ID NO: 1) |
| Chothia/Ab M/Kabat combinatio n | GFTFSRHGMT (SEQ ID NO: 41) | EINIPGDDVDYDISVKG (SEQ ID NO: 42) | AGENMRLVT (SEQ ID NO: 1) |

For example, by dividing the amino acid sequences shown in SEQ ID NO: 24 or SEQ ID NO: 25 using the method in Table 2, the isolated antigen binding protein in the present application may contain CDRs as shown in the following table.

**Table 2. CDR sequences obtained by dividing the amino acid sequences shown in SEQ ID NO: 24 or SEQ ID NO: 25 in different ways**

| **Method** | **CDR1** | **CDR2** | **CDR3** |
|---|---|---|---|
| Chothia | GFTISST (SEQ ID NO: 16) | NIPGTD (SEQ ID NO: 15) | QGANLRQGV (SEQ ID NO: 14) |
| AbM | GFTISSTGMT (SEQ ID NO: 45) | DINIPGTDTD (SEQ ID NO: 44) | QGANLRQGV (SEQ ID NO: 14) |
| Kabat | STGMT (SEQ ID NO: 47) | DINIPGTDTDYDISVKG (SEQ ID NO: 46) | QGANLRQGV (SEQ ID NO: 14) |
| Chothia/Ab M/Kabat combinatio n | GFTISSTGMT (SEQ ID NO: 45) | DINIPGTDTDYDISVKG (SEQ ID NO: 46) | QGANLRQGV (SEQ ID NO: 14) |

For example, by dividing the amino acid sequences shown in SEQ ID NO: 37 or SEQ ID NO: 38 using the method in Table 3, the isolated antigen binding protein in the present application may comprise CDRs as shown in the following table.

**Table 3. CDR sequences obtained by dividing the amino acid sequences shown in SEQ ID NO: 37 or SEQ ID NO: 38 in different ways**

| **Method** | **CDR1** | **CDR2** | **CDR3** |
|---|---|---|---|
| Chothia | GSTLDSY (SEQ ID NO: 28) | SRSGAS (SEQ ID NO: 27) | YRNFGNFCVMGR YEYDE (SEQ ID NO: 26) |
| AbM | GSTLDSYTVA (SEQ ID NO: 49) | CASRSGASTN (SEQ ID NO: 48) | YRNFGNFCVMGR YEYDE (SEQ ID NO: 26) |
| Kabat | SYTVA (SEQ ID NO: 51) | CASRSGASTNYANSVKG (SEQ ID NO: 50) | YRNFGNFCVMGR YEYDE (SEQ ID NO: 26) |
| Chothia/Ab M/Kabat combinatio n | GSTLDSYTVA (SEQ ID NO: 49) | CASRSGASTNYANSVKG (SEQ ID NO: 50) | YRNFGNFCVMGR YEYDE (SEQ ID NO: 26) |

In the present application, the isolated antigen binding protein may comprise VHH. In some cases, the CDR3 of the VHH may comprise an amino acid sequence shown in SEQ ID NO: 1, the CDR2 of the VHH can comprise an amino acid sequence shown in SEQ ID NO: 2, and the CDR1 of the VHH can comprise an amino acid sequence shown in SEQ ID NO: 3. In some cases, the CDR3 of the VHH can comprise an amino acid sequence shown in SEQ ID NO: 14, the CDR2 of the VHH can comprise an amino acid sequence shown in SEQ ID NO: 15, and the CDR1 of the VHH can comprise an amino acid sequence shown in SEQ ID NO: 16. In some cases, the CDR3 of the VHH can comprise an amino acid sequence shown in SEQ ID NO: 26, the CDR2 of the VHH can comprise an amino acid sequence shown in SEQ ID NO: 27, and the CDR1 of the VHH can comprise an amino acid sequence shown in SEQ ID NO: 28.

In the present application, the isolated antigen binding protein can comprise H-FR1, and the C-terminus of H-FR1 can be directly or indirectly linked to the N-terminus of HCDR1. In some cases, the H-FR1 may comprise an amino acid sequence shown in SEQ ID NO: 54.

In some cases, the H-FR1 may comprise an amino acid sequence shown in SEQ ID NO: 4. In some cases, the H-FR1 may comprise an amino acid sequence shown in SEQ ID NO: 8. In some cases, the H-FR1 may comprise an amino acid sequence shown in SEQ ID NO: 17. In some cases, the H-FR1 may comprise an amino acid sequence shown in SEQ ID NO: 29. In some cases, the H-FR1 may comprise an amino acid sequence shown in SEQ ID NO: 33.

In the present application, the isolated antigen binding protein may comprise H-FR2, which can be located between HCDR1 and HCDR2. In some cases, the H-FR2 may comprise an amino acid sequence shown in SEQ ID NO: 55. In some cases, the H-FR2 may comprise an amino acid sequence shown in SEQ ID NO: 56.

In some cases, the H-FR2 may comprise an amino acid sequence as shown in SEQ ID NO: 5. In some cases, the H-FR2 may comprise an amino acid sequence as shown in SEQ ID NO: 9. In some cases, the H-FR2 may comprise an amino acid sequence as shown in SEQ ID NO: 18. In some cases, the H-FR2 may comprise an amino acid sequence as shown in SEQ ID NO: 21. In some cases, the H-FR2 may comprise an amino acid sequence as shown in SEQ ID NO: 30. In some cases, the H-FR2 may comprise an amino acid sequence as shown in SEQ ID NO: 34.

In this application, the isolated antigen binding protein may comprise H-FR3, which can be located between HCDR2 and HCDR3. In some cases, the H-FR3 may comprise an amino acid sequence as shown in SEQ ID NO: 57. In some cases, the H-FR3 may comprise an amino acid sequence as shown in SEQ ID NO: 58.

In some cases, the H-FR3 may comprise an amino acid sequence as shown in SEQ ID NO: 6. In some cases, the H-FR3 may comprise an amino acid sequence as shown in SEQ ID NO: 10. In some cases, the H-FR3 may comprise an amino acid sequence as shown in SEQ ID NO: 19. In some cases, the H-FR3 may comprise an amino acid sequence as shown in SEQ ID NO: 22. In some cases, the H-FR3 may comprise an amino acid sequence as shown in SEQ ID NO: 31. In some cases, the H-FR3 may comprise an amino acid sequence as shown in SEQ ID NO: 35.

In the present application, the isolated antigen binding protein may comprise H-FR4, and the N-terminus of H-FR4 may be linked to the C-terminus of HCDR3. In some cases, the H-FR4 may comprise an amino acid sequence as shown in SEQ ID NO: 59.

In some cases, the H-FR4 may comprise an amino acid sequence as shown in SEQ ID NO: 7. In some cases, the H-FR4 may comprise an amino acid sequence as shown in SEQ ID NO: 11. In some cases, the H-FR4 may comprise an amino acid sequence as shown in SEQ ID NO: 20. In some cases, the H-FR4 may comprise an amino acid sequence as shown in SEQ ID NO: 23. In some cases, the H-FR4 may comprise an amino acid sequence as shown in SEQ ID NO: 32. In some cases, the H-FR4 may comprise an amino acid sequence as shown in SEQ ID NO: 36.

In the present application, the isolated antigen binding protein may comprise H-FR1, H-FR2, H-FR3, and H-FR4. For example, the H-FR1 may comprise an amino acid sequence shown in SEQ ID NO: 54, the H-FR2 may comprise an amino acid sequence shown in SEQ ID NO: 55 or SEQ ID NO: 56, the H-FR3 may comprise an amino acid sequence shown in SEQ ID NO: 57 or SEQ ID NO: 58, and the H-FR4 may comprise an amino acid sequence shown in SEQ ID NO: 59.

In some cases, the isolated antigen binding protein may comprise H-FR1, H-FR2, H-FR3, and H-FR4, wherein the H-FR1 may comprise an amino acid sequence shown in any of SEQ ID NO: 4, SEQ ID NO: 8, SEQ ID NO: 17, SEQ ID NO: 29, and SEQ ID NO: 33, the H-FR2 may comprise an amino acid sequence shown in any of SEQ ID NO: 5, SEQ ID NO: 9, SEQ ID NO: 18, SEQ ID NO: 21, SEQ ID NO: 30, and SEQ ID NO: 34, the H-FR3 may comprise an amino acid sequence shown in any of SEQ ID NO: 6, SEQ ID NO: 10, SEQ ID NO: 19, SEQ ID NO: 22, SEQ ID NO: 31, and SEQ ID NO: 35, and the H-FR4 may comprise an amino acid sequence shown in any of SEQ ID NO: 7, SEQ ID NO: 11, SEQ ID NO: 20, SEQ ID NO: 23, SEQ ID NO: 32, and SEQ ID NO: 36.

In the present application, the isolated antigen binding protein may comprise H-FR1, H-FR2, H-FR3, and H-FR4. For example, the H-FR1, H-FR2, H-FR3, and H-FR4 of the isolated antigen binding proteins may sequentially comprise the amino acid sequences shown in SEQ ID NO: 4, SEQ ID NO: 5, SEQ ID NO: 6, and SEQ ID NO: 7, respectively. For example, the H-FR1, H-FR2, H-FR3, and H-FR4 of the isolated antigen binding proteins may sequentially comprise the amino acid sequences shown in SEQ ID NO: 8, SEQ ID NO: 9, SEQ ID NO: 10, and SEQ ID NO: 11, respectively. For example, the H-FR1, H-FR2, H-FR3, and H-FR4 of the isolated antigen binding proteins may sequentially comprise the amino acid sequences shown in SEQ ID NO: 17, SEQ ID NO: 18, SEQ ID NO: 19, and SEQ ID NO: 20, respectively. For example, the H-FR1, H-FR2, H-FR3, and H-FR4 of the isolated antigen binding proteins may sequentially comprise the amino acid sequences shown in SEQ ID NO: 8, SEQ ID NO: 21, SEQ ID NO: 22, and SEQ ID NO: 23, respectively. For example, the H-FR1, H-FR2, H-FR3, and H-FR4 of the isolated antigen binding proteins may sequentially comprise the amino acid sequences shown in SEQ ID NO: 29, SEQ ID NO: 30, SEQ ID NO: 31, and SEQ ID NO: 32, respectively. For example, the H-FR1, H-FR2, H-FR3, and H-FR4 of the isolated antigen binding proteins may sequentially comprise the amino acid sequences shown in SEQ ID NO: 33, SEQ ID NO: 34, SEQ ID NO: 35, and SEQ ID NO: 36, respectively.

In the present application, the isolated antigen binding protein may comprise VH. In some cases, the VH may comprise an amino acid sequence as shown in SEQ ID NO: 60. In some cases, the VH may comprise an amino acid sequence as shown in SEQ ID NO: 61.

In some cases, the VH may comprise an amino acid sequence as shown in SEQ ID NO: 12. In some cases, the VH may comprise an amino acid sequence as shown in SEQ ID NO: 13. In some cases, the VH may comprise an amino acid sequence as shown in SEQ ID NO: 24. In some cases, the VH may comprise an amino acid sequence as shown in SEQ ID NO: 25. In some cases, the VH may comprise an amino acid sequence as shown in SEQ ID NO: 37. In some cases, the VH may comprise an amino acid sequence as shown in SEQ ID NO: 38.

In the present application, the isolated antigen binding protein may comprise VHH. In some cases, the VHH may comprise an amino acid sequence as shown in SEQ ID NO: 60. In some cases, the VHH may comprise an amino acid sequence as shown in SEQ ID NO: 61.

In some cases, the VHH may comprise an amino acid sequence as shown in SEQ ID NO: 12. In some cases, the VHH may comprise an amino acid sequence as shown in SEQ ID NO: 13. In some cases, the VHH may comprise an amino acid sequence as shown in SEQ ID NO: 24. In some cases, the VHH may comprise an amino acid sequence as shown in SEQ ID NO: 25. In some cases, the VHH may comprise an amino acid sequence as shown in SEQ ID NO: 37. In some cases, the VHH may comprise an amino acid sequence as shown in SEQ ID NO: 38.

In the present application, the isolated antigen binding protein may comprise an antibody heavy chain constant region. In certain embodiments, the heavy chain constant region of an antibody may be derived from the heavy chain constant region of any immunoglobulin, including IgM, IgD, IgG, IgA, and IgE. For example, the heavy chain constant region of an antibody may be derived from human IgG heavy chain constant region. In some embodiments, the heavy chain constant region of the immunoglobulin includes its mutant. In certain embodiments, the heavy chain constant region of an antibody may be derived from the heavy chain constant region of any human IgG1-4. In some embodiments, the isolated antigen binding protein may be derived from human IgG1 heavy chain constant region. For example, the constant region of the human IgG1 heavy chain may comprise an amino acid sequence as shown in SEQ ID NO: 39. For example, the isolated antigen binding protein may comprise an amino acid sequence as shown in SEQ ID NO: 62. For example, the isolated antigen binding protein may comprise an amino acid sequence as shown in SEQ ID NO: 63. For example, the isolated antigen binding protein may comprise an amino acid sequence as shown in SEQ ID NO: 64. For example, the isolated antigen binding protein may comprise an amino acid sequence as shown in SEQ ID NO: 68. For example, the isolated antigen binding protein may comprise an amino acid sequence as shown in SEQ ID NO: 69. For example, the isolated antigen binding protein may comprise an amino acid sequence as shown in SEQ ID NO: 70.

In the present application, the isolated antigen binding protein may comprise antibodies or their antigen binding fragments.

In certain embodiments, the antigen binding fragments may include Fab, Fab', Fv fragments, F(ab')₂, F(ab)₂, scFv, bs-Fv, di-scFv, and/or dAb.

In the present application, the isolated antigen binding protein may comprise VHH. In some cases, the VHH may comprise an amino acid sequence as shown in SEQ ID NO: 60. In some cases, the VHH may comprise an amino acid sequence as shown in SEQ ID NO: 61.

In some cases, the VHH may comprise an amino acid sequence as shown in SEQ ID NO: 12. In some cases, the VHH may comprise an amino acid sequence as shown in SEQ ID NO: 13. In some cases, the VHH may comprise an amino acid sequence as shown in SEQ ID NO: 24. In some cases, the VHH may comprise an amino acid sequence as shown in SEQ ID NO: 25. In some cases, the VHH may comprise an amino acid sequence as shown in SEQ ID NO: 37. In some cases, the VHH may comprise an amino acid sequence as shown in SEQ ID NO: 38.

In the present application, the antibodies may include monoclonal antibodies, chimeric antibodies, humanized antibodies, and/or fully human antibodies.

For example, the VH of the humanized antigen binding protein may comprise an amino acid sequence shown in SEQ ID NO: 13. For example, the VH of the humanized antigen binding protein may comprise an amino acid sequence shown in SEQ ID NO: 25. For example, the VH of the humanized antigen binding protein may comprise an amino acid sequence shown in SEQ ID NO: 38.

In the present application, the isolated antigen binding protein may include a tetravalent antigen binding protein and/or a tetravalent antigen binding protein Fc fusion protein. For example, the variable region coding sequences of two anti-alpha toxin antigen binding proteins can be linked in series (such as two huA3 molecules or two huG1 molecules or one huA3 molecule and one huG1 molecule as described herein; such as two A3 molecules or two G1 molecules or one A3 molecule and one G1 molecule as described herein) and fused with a human IgG1 Fc fragment. For example, the C therminal of the first VHH may be connected with the N terminus of the second VHH, and the C terminus of the second VHH may be connected with the N terminus of the Fc fragment to obtain a bivalent Fc fusion protein, and the bivalent Fc fusion protein can be subcloned in a plasmid and transfected to a cell for recombinant expression to obtain the tetravalent antigen binding protein of the present application. For example, the isolated antigen binding protein may comprise huA3-huG1-Fc fusion proteins binding with antigen. For example, the isolated antigen binding protein may comprise huA3-huA3-Fc fusion proteins binding with antigen. For example, the isolated antigen binding protein may comprise huG1-huG1-Fc fusion proteins binding with antigen. For example, the isolated antigen binding protein may comprise an amino acid sequence as shown in SEQ ID NO: 65. For example, the isolated antigen binding protein may comprise an amino acid sequence as shown in SEQ ID NO: 66. For example, the isolated antigen binding protein may comprise an amino acid sequence as shown in SEQ ID NO: 67.

In addition, it should be noted that the isolated antigen binding protein described herein may comprise heavy chain and/or light chain sequences with one or more conserved sequence modifications. The so-called "conserved sequence modification" refers to an amino acid modification that will not significantly affect or change the binding characteristics of antibodies. Such conserved modifications include substitution, addition, and deletion of amino acids. The modification may be introduced into the isolated antigen binding protein described herein through standard technologies known in the art, such as point mutation and PCR mediated mutation. Conserved amino acid substitution refers to the replacement of amino acid residues with amino acid residues with similar side chains. Amino acid residues with similar side chains are known in the field. These amino acid residue groups include those with a basic side chain (e.g., lysine, arginine, histidine), an acidic side chain (e.g., aspartic acid, glutamic acid), a uncharged polar side chain (e.g., glycine, asparagine, glutamine, serine, threonine, tyrosine, cysteine, tryptophan), a non-polar side chains (e.g., alanine, valine, leucine, isoleucine, proline, phenylalanine, methionine), a β-branched side chain (e.g., threonine, valine, isoleucine) and an aromatic side chain (e.g., tyrosine, phenylalanine, tryptophan, histidine). In certain embodiments, one or more amino acid residues in the CDR region of the isolated antigen binding protein described herein can be replaced with another amino acid residues in the same side chain group. Those skilled in the art know that some conserved modification of sequence will not destroy antigen binding. For details, see, for example, Brummell et. al., (1993) Biochem 32:1180-8; de Wildt et. al., (1997) Prot. Eng. 10:835-41; Komisarov et. al., (1997) J. Biol. Chem. 272:26864-26870; Hall et. al., (1992) J. Immunol. 149:1605-12; Kelley and O'Connell (1993) Biochem. 32:6862-35; Adib Conquy et. al., (1998) Int. Immunol. 10:341-6 and Beers et. al., (2000) Clin. Can. Res. 6:2835-43.

The *Staphylococcus aureus* alpha toxin antigen binding protein described herein may be identified, screened, or characterized by various known assays in the art.

For example, known methods such as ELISA (ELISA), immunoblotting (e.g., Western blotting), flow cytometry (e.g., FACS), immunohistochemistry, immunofluorescence, etc. may be used to test the antigen binding activity of the antigen binding protein or fusion protein of the present application.

In the present application, the isolated antigen binding protein may specifically bind to *Staphylococcus aureus* alpha toxin. In some embodiments, the binding of the isolated antigen binding protein to *Staphylococcus aureus* alpha toxin may be detected by ELISA method. For example, the antigen-binding protein described herein may bind to *Staphylococcus aureus* alpha toxin at an EC₅₀ value of less than or equal to approximately 0.010 µg/mL, less than or equal to approximately 0.009 µg/mL, less than or equal to approximately 0.008 µg/mL, less than or equal to approximately 0.007 µg/mL, less than or equal to approximately 0.006 µg/mL, less than or equal to approximately 0.005 µg/mL, less than or equal to approximately 0.004 µg/mL, less than or equal to approximately 0.003 µg/mL, less than or equal to approximately 0.002 µg/mL and less than or equal to approximately 0.001 µg/mL.

In the present application, the isolated antigen binding protein may neutralize the biological activity of *Staphylococcus aureus* alpha toxin. For example, it may be achieved by adding rabbit red blood cells to the *Staphylococcus aureus* alpha toxin and antigen binding protein and detecting their anti-hemolytic activity with an enzyme-linked immunosorbent assay. For example, the antigen binding protein in the present application may be used in an amount of less than or equal to about 200ng, less than or equal to about 100ng, less than or equal to about 75ng, less than or equal to about 60ng, less than or equal to about 50ng, less than or equal to about 45ng, less than or equal to about 40ng, and less than or equal to about 35ng to completely neutralize 18.75ng recombinant *Staphylococcus aureus* alpha toxin.

In the present application, the isolated antigen binding protein may prevent and/or treat diseases and/or conditions and their complications. In some embodiments, the disease and/or condition and complication thereof may be caused or mediated by *Staphylococcus aureus.* In some embodiments, the disease and/or condition and complication thereof may include sepsis and/or bacteremia.

### Polypeptide molecules, nucleic acid molecules, vectors, cells, immunoconjugates and pharmaceutical compositions

In another aspect, the present application provides polypeptide molecules that may comprise the isolated antigen binding proteins described herein.

In certain embodiments, the polypeptide molecule may comprise a fusion protein. In certain embodiments, the polypeptide molecule may be a fusion protein.

In certain embodiments, the polypeptide molecule comprises a fusion protein consisting of two or more isolated antigen binding proteins as described herein and a heavy chain constant region of an antibody.

For example, the polypeptide molecule may comprise two antigen binding domains and a constant region of an antibody heavy chain. The first antigen-binding domain may include any of the isolated antigen-binding proteins in the present application, and the second antigen-binding domain may include any of the isolated antigen-binding proteins in the present application.

In certain embodiments, the polypeptide molecule may comprise an amino acid sequence shown in any of SEQ ID NO: 65, SEQ ID NO: 66, and SEQ ID NO: 67.

In one aspect, the present application provides an isolated nucleic acid molecule that may encode the isolated antigen binding protein described herein. For example, it can be produced or synthesized by the following methods: (i) *in vitro* amplification, such as polymerase chain reaction (PCR) amplification; (ii) cloning and recombination; (iii) purification, for example, by enzyme digestion and gel electrophoresis fractionation; or (iv) synthesis, such as by chemical synthesis.

In another aspect, the present application provides a vector that may comprise the nucleic acid molecules described herein. In addition, the vector may also comprise other genes, such as labelling genes that allow selection of the vector in appropriate host cells and under appropriate conditions. In addition, the vector may also include an expression control element that allows the coding region to be correctly expressed in an appropriate host. Such control element is familiar to those skilled in the art, for example, it may comprise promoter, ribosome-binding site, enhancer, and other control elements that regulate gene transcription or mRNA translation. The vector can transform, transduce or transfect a host cell, so that the genetic material elements carried by the vector can be expressed in the host cell. The vectors may include, for example, plasmids, cosmids, viruses, bacteriophages, or other vectors commonly used in genetic engineering. For example, the vector is an expression vector. In addition, the vector may also include components that assist it in entering cells, such as viral particles, liposomes, or protein coats, but not limited to these substances.

In another aspect, the present application provides a cell that may comprise the nucleic acid molecule, or the vector described herein. In certain embodiments, each or every host cell may comprise one or more nucleic acid molecules or vectors described herein. In certain embodiments, each or every host cell may comprise multiple number of (e.g., two or more number of) or multiple (e.g., two or more) nucleic acid molecules or vectors described herein. For example, the vector described herein may be introduced into host cells, such as eukaryotic cells, such as cells from plants, fungi, or yeast cells. In some embodiments, the cells may be bacterial cells (e.g., *Escherichia coli),* yeast cells or other eukaryotic cells, such as COS cells, Chinese hamster ovary (CHO) cells, CHO-K1 cells, LNCAP cells, HeLa cells, 293T cells, COS-1 cells, SP2/0 cells, NS0 cells or myeloma cells. The vector described herein may be introduced into the host cells through methods known in the art, such as electroporation, lipofectine transfection, lipofectamine transfection, etc.

In another aspect, the present application also provides an immunoconjugate, which may comprise the isolated antigen binding protein described herein.

In some embodiments, the isolated antigen binding proteins or their fragments described herein may be linked to another reagent, such as a chemotherapeutic agent, toxin, immunotherapeutic agent, imaging probe, spectroscopic probe, etc. The linkage may be through one or more covalent bond, or non-covalent interactions, and may include chelation. Several linkers may be used (which are known in the art) to form immunoconjugates. In addition, immunoconjugates can be provided in the form of fusion proteins, which can be expressed by polynucleotides encoding immunoconjugates. The immunoconjugate may also include, for example, an antibody drug conjugate (ADC). Suitable drugs may comprise cytotoxin, alkylating agent, DNA groove binding molecule, DNA intercalating agent, DNA cross-linking agent, histone deacetylase inhibitor, nuclear output inhibitor, proteasome inhibitor, Topoisomerase I or II inhibitor, heat shock protein inhibitor, tyrosine kinase inhibitor, antibiotic and antimitotic agent. In ADC, antibodies and therapeutic agents can be cross-linked through linkers that can be cleaved, such as peptide linkers, disulfide linkers, or hydrazone linkers.

In another aspect, the present application also provides a pharmaceutical composition, which may include the isolated antigen binding protein, polypeptide molecule, immunoconjugate, nucleic acid molecule, vector and/or cell described herein, as well as optionly a pharmaceutically acceptable vehicle.

In certain embodiments, the pharmaceutical composition may also include one or more (pharmaceutically effective) adjuvants, stabilizers, excipients, diluents, solubilizers, surfactants, emulsifiers, and/or preservatives. The acceptable components of the composition are preferably non-toxic to the recipient at the dosage and concentration used. The pharmaceutical compositions of the present invention include but are not limited to liquid, frozen, and lyophilized compositions.

In certain embodiments, the pharmaceutical composition may also comprise more than one active compound, typically those with complementary activity that do not adversely affect each other. The type and effective amount of such drugs may depend on, for example, the amount and type of antagonists present in the formulation, as well as the clinical parameters of the subjects.

In certain embodiments, the pharmaceutically acceptable vehicle may include any and all solvents, dispersants, coatings, isotopes, and absorption delay agents compatible with drug administration, typically safe and non-toxic.

In certain embodiments, the pharmaceutical composition may include parenteral, percutaneous, intracavitary, intra-arterial, intrathecal, and/or intranasal administration or direct injection into tissues. For example, the pharmaceutical composition can be administered to patients or subjects through infusion or injection. In certain embodiments, the administration of the pharmaceutical composition can be carried out in different ways, such as intravenous, intraperitoneal, subcutaneous, intramuscular, local, or dermal administration. In certain embodiments, the pharmaceutical composition may be administered continuously. The uninterrupted (or continuous) administration may be achieved through a small pump system worn by the patient to measure the therapeutic agent flowing into the patient's body, as described in WO2015/036583.

### Methods of preparation

In another aspect, the present application provides a method for preparing the isolated antigen binding protein. The method may comprise culturing the host cell described herein under the conditions for expressing the antigen binding protein. For example, by using appropriate culture media, appropriate temperature, and culture time, these methods may be understood by those skilled in the art.

Any method suitable for producing monoclonal antibodies can be used to produce the antigen binding protein of the present application. For example, linked or native *Staphylococcus aureus* alpha toxin or their fragments can be used to immunize animals. Suitable immunization methods may be used, including adjuvants, immunostimulants, repeated booster immunization, and one or more pathways may be used. In some embodiments, immunized alpaca peripheral blood lymphocytes may be extracted, cell nucleic acid fragments may be extracted and cloned into vectors, and isolated antigen binding proteins against *Staphylococcus aureus* alpha toxin may be screened and enriched by a phage surface display system.

Any suitable form of *Staphylococcus aureus* alpha toxin may be used as immunogens (antigens) to produce non-human antibodies specific for *Staphylococcus aureus* alpha toxin, and to screen the biological activity of the antibodies. For example, the trigger immunogen can be full-length *Staphylococcus aureus* alpha toxin, including natural homodimers or peptides containing single/multiple epitopes. Immunogens can be used alone or in combination with one or more known immunogenic enhancers in the art.

### Method and use

In another aspect, the present application provides use of the isolated antigen binding protein, polypeptide molecule, nucleic acid molecule, vector, cell, immunoconjugate, and/or pharmaceutical combination in the manufacture of a medicament for prevention and/or treatment of diseases and/or conditions and their complications.

In another aspect, the present application also provides a method for preventing and/or treating diseases and/or conditions and their complications, which may comprise administering the isolated antigen binding protein, polypeptide molecule, nucleic acid molecule, vector, cell, immunoconjugate, and/or pharmaceutical composition described herein to a subject in need.

In the present application, the isolated antigen binding protein, polypeptide molecule, nucleic acid molecule, vector, cell, immunoconjugate, and/or pharmaceutical composition described herein can be used alone or in combination with other drugs to prevent and/or treat diseases and/or conditions. In certain embodiments, the other drugs may be any currently known drug with antibacterial effects. The diseases and/or conditions may be caused by *Staphylococcus aureus* infection or related diseases caused by other infections.

In the present application, the administration may be carried out in different ways, such as intravenous, intratumoral, intraperitoneal, subcutaneous, intramuscular, local or intradermal administration.

In another aspect, the isolated antigen binding protein, polypeptide molecule, nucleic acid molecule, vector, cell, immunoconjugate, and/or pharmaceutical composition described herein may be used for preventing and/or treating diseases and/or conditions.

In the present application, the diseases and/or conditions may be caused or mediated by *Staphylococcus aureus.*

In the present application, the diseases and/or conditions can be complications of the diseases and/or consitions caused or mediated by *Staphylococcus aureus.*

In the present application, the diseases and/or conditions and their complications include bacterial infections.

In the present application, the diseases and/or conditions and their complications include bacteremia and/or sepsis.

In some cases, the isolated antigen binding protein, polypeptide molecule, nucleic acid molecule, vector, cell, immunoconjugate, and/or pharmaceutical composition described herein may be used alone or in combination with other drugs.

In one aspect, the present application also provides a method for detecting *Staphylococcus aureus* alpha toxin in a sample, which comprises administering the isolated antigen binding protein, polypeptide molecule, nucleic acid molecule, vector, cell, immunoconjugate, and/or pharmaceutical composition.

In some cases, the method for detecting *Staphylococcus aureus* alpha toxin in a sample may be an *in vitro* method. For example, the isolated antigen binding protein described herein is contacted with an *ex vivo* sample to detect the presence and/or content of *Staphylococcus aureus* alpha toxin in the sample. In some cases, the method for detecting *Staphylococcus aureus* alpha toxin in a sample is not for therapeutic purposes. In some cases, the method for detecting *Staphylococcus aureus* alpha toxin in a sample is not a diagnostic method.

In another aspect, the present application also provides a reagent or kit for detecting *Staphylococcus aureus* alpha toxin in a sample comprising the isolated antigen binding protein, polypeptide molecule, nucleic acid molecule, vector, cell, immunoconjugate, and/or pharmaceutical composition.

In another aspect, the present application also provides use of the isolated antigen binding protein, the polypeptide molecule, the nucleic acid molecule, the vector, the cell, the immunoconjugate and/or the pharmaceutical composition in the manufacture of a kit for detecting the presence and/or content of *Staphylococcus aureus* alpha toxin in a sample.

Without being limited by any theory, the following examples are only intended to illustrate the antigen binding protein, preparation method, and use of the present application, and are not intended to limit the scope of the present invention.

### Examples

### Example 1. Immunization of alpaca with recombinantly expressed Staphylococcus aureus alpha toxin

One alpaca was immunized with the prepared *Staphylococcus aureus* alpha toxin and receive multiple subcutaneous immunizations four times. The immunization protocol was shown in Table 4.

**Table 4. Immunization protocol of alpaca**

| | |
|---|---|
| First immunization | 5ml blood was collected as negative serum before immunization; the adjuvant was mixed with antigen (1 mg) at a ratio of 1:1, emulsified, and then injected at multiple points internally and subcutaneously |
| Second | the antigen (2 mg) was mixed with the adjuvant at a ratio of 1:1, immunization emulsified and injected subcutaneously at multiple points |
| Third immunization | the antigen (2 mg) was mixed with the adjuvant at a ratio of 1:1, emulsified and injected subcutaneously at multiple points. Serum was collected one week after third immunization for titer detection |
| Boost | the antigen (2mg), jugular vein injection |
| | immunization |
| | 50ml peripheral blood was collected 3 days after boost immunization for single B cell antibody sequence cloning |

### Example 2. Construction and screening of a library of Staphylococcus aureus alpha toxin antigen binding proteins

100ml peripheral blood lymphocytes from immunized alpaca was extracted, and the total RNA of cells was extracted with a kit from Qigen. The RNA was reverse transcribed into cDNA using a reverse transcription kit (Qigen). The nucleic acid fragments encoding variable region of the antibodies wer amplified by PCR. PCR products and clone vector pComb3Xss were cleaved with restriction endonuclease SfilI (Takara), and T4 ligase was used to connect the clone vector to the phage vector pComb3Xss. The product was then electro-transferred into the *Escherichia coli* completent cell Top 10.

### Example 3. Selection for antigen binding proteins against Staphylococcus aureus alpha toxin

The alpha toxin fusion protein was used to coat a 96 well plate and placed overnight at 4 °C. On the second day, it was blocked with 5% BSA and then added 100 µl phage. After incubation at room temperature for 1 hour, it was washed with PBST to remove unbound phages. Finally, the phage specifically bound to alpha toxin was dissociated with triethylamine and used to infect *Escherichia coli* TG1 in logarithmic phase growth, the phage was produced and purified for the next round of screening. The same screening process was repeated for 3-4 rounds. As a result, positive clones were enriched to screen alpha toxin specific antibodies in the antibody library using phage display.

### Example 4. Screening of specific single positive clones using phage-based enzyme-linked immunosorbent assay (ELISA)

The obtained *Staphylococcus aureus* alpha toxin binding positive phages were used to infect *Escherichia coli* coated plates. Subsequently, 210 single colonies were picked and cultured separately to produce and purify phages. The alpha toxin fusion protein was coated on a 96 well plate at 4 °C overnight, the obtained sample phage (with blank phage as control group) was added, and the reaction was conducted at room temperature for 1 hour. After washing, the mouse anti-HA labeled antibody was added and reacted at room temperature for 1 hour. After washing, the goat anti-mouse HRP antibody (Beijing Dingguo Biotechnology Co., Ltd.) was added and reacted at room temperature for 1 hour. After washing, TMB development solution was added and the absorption value at a wavelength of 405nm was read. When the OD value of a sample well was more than three times of that of the control well, the sample well was labeled as a positive clone well. The bacteria in the positive clone well were transferred to LB liquid containing 100 µl Ampicillin for culture to extract plasmid and to sequence. Finally, the antigen binding protein sequence was obtained.

The sequence of the antigen binding protein was determined as follows:
A3 SEQ ID NO: 12 
QVQLVESGGGLVQAGGSLTLSCVASGFTFSRHGMTWVRQAPGKELDYVGEINIPGDD
VDYDISVKGRFTISRDNAKNTVYLHMTNLKVEDTATYWCAQAGENMRLVTRRQGTQVTV
ST
G1 SEQ ID NO: 24 
QVQLVESGGDLVQPGGSLTLACEASGFTISSTGMTWVRQAPGKELDWVGDINIPGTDT
DYDISVKGRFTISKDHAKNTVYLQMTSLRPEDTAIYYCAKQGANLRQGVQSPGTLVTVSS
 F6 SEQ ID NO: 37

QVQLVESGGGLAQPGGSLRLSCEASGSTLDSYTVAWFRQAPGKEREGVSCASRSGAST
NYANSVKGRFTISRDNAKNTVYLQMNSLKPEDTGVYYCAAYRNFGNFCVMGRYEYDEWG
QGTQVTVSS

Wherein, the underlined part represents the CDR regions generated using the Chothia numbering scheme.

### Example 5. Expression and purification of the antigen binding protein fused with Fc in Expi293 cells

The variable region coding sequences of antigen-binding proteins A3, G1, and F6 obtained from sequencing analysis were fused with the Fc fragment of human immunoglobulin γ1 (IgG1) and subcloned into expression vector PCDNA3.4. Expi293 cells were transfected with PEI for recombinant expression. After 7 days, the cell supernatant was collected and purified using ProteinA magnetic beads. The antigen binding protein with a purity of over 95% was collected, and A3-Fc fusion proteins binding with antigen, G1-Fc fusion proteins binding with antigen, and F6-Fc fusion proteins binding with antigen were obtained. The amino acid sequence of A3-Fc fusion proteins binding with antigen was shown in SEQ ID NO: 62, the amino acid sequence of G1-Fc fusion proteins binding with antigen was shown in SEQ ID NO: 63, and the amino acid sequence of F6-Fc fusion proteins binding with antigen was shown in SEQ ID NO: 64.

### Example 6. Detection of the binding of the antigen binding protein with Staphylococcus aureus alpha toxin by ELISA

*Staphylococcus aureus* alpha toxin protein was diluted the with PBS buffer to 1 µg/ml, coated with 100 µl per well and incubated overnight at 4°C on a 96 well plate (Thermo); on the next day, the 96 well plate was taken out and washed with PBST (containing 0.5% PBS), after it was socked for 1 minute each time, the residual moisture was thoroughly removed by spin. 200 µl PBST containing 5% BSA was added to the wells and sealed at 37°C for 1 hour; then the plate was washed with PBST and the residual moisture in wells was thoroughly removed by spin. 100 µl of the test sample was added to the 96 well plate and incubated at 4°C overnight. After the 96 well plate was removed and washed with PBST, 100 µl of goat anti-human IgG secondary antibody (Thermo, 31413, Goat anti-human IgG Fc Cross-Adsorbed Secondary Antibody, HRP) was added to each well, and incubated at 37°C for 1 hour. It was washed 5 times with PBST, 100 µl of Substrate Solution (Invitrogen) was added to each well and incubated at 37°C for 10 minutes; 1mol of 50 µl sulfuric acid was added to each well to terminate the reaction, and then the absorbance at a wavelength of 450 nm was measured using a microplate reader (Multiskcin FC, Thermo). Human IgG was used as a negative control.

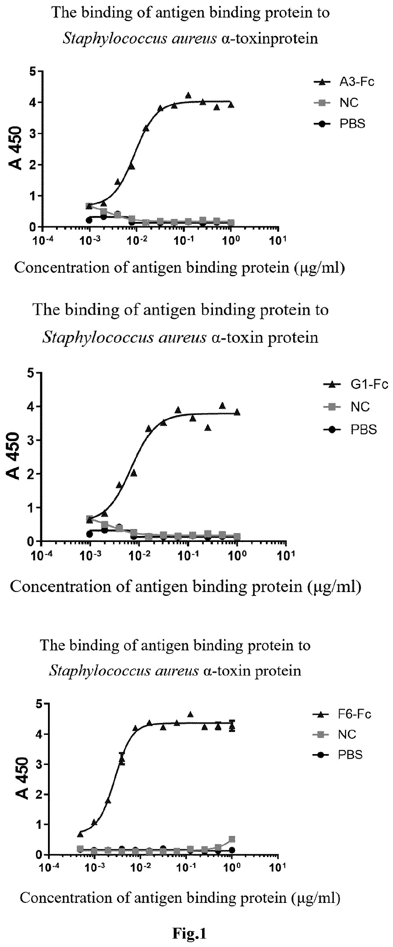

The results of binding detection of A3-Fc fusion proteins binding with antigen, G1-Fc fusion proteins binding with antigen, F6-Fc fusion proteins binding with antigen with *Staphylococcus aureus* alpha toxin were shown in Fig. 1. The results showed that the above three antigen binding proteins had strong affinity with *Staphylococcus aureus* alpha toxin, wherein the EC₅₀ value of the A3-Fc fusion proteins binding with antigen was 0.009 µg/ml, the EC₅₀ value of G1-Fc fusion proteins binding with antigen was 0.007 µg/ml, and the EC₅₀ value of F6-Fc fusion proteins binding with antigen was 0.003 µg/ml.

### Example 7. Evaluation of neutralization of hemolytic activity of Staphylococcus aureus alpha toxin by the antigen binding protein

AR-301 is a fully human antibody drug (Salvecin, tosatoxumab)that targets alpha toxin developed by Aridis Pharmaceuticals. Evaluation of neutralization of the hemolytic activity of *Staphylococcus aureus* alpha toxin by AR301 was conducted by rabbit erythrocyte toxin model induced by alpha toxin: 18.75ng recombinant *Staphylococcus aureus* alpha toxin protein and antigen binding protein or AR-301 were mixed at a certain ratio and added to a 96 well plate (Thermo), incubated at 37°C for 10 minutes, 5% rabbit red blood cells were added, and incubated at 37°C for 1 hour. The sample was centrifuged at 3000 rpm for 5 minutes, and the anti-hemolytic activity was determined by detecting the absorbance at a wavelength of 405 nm using a microplate reader (Multiskcin FC, Thermo).

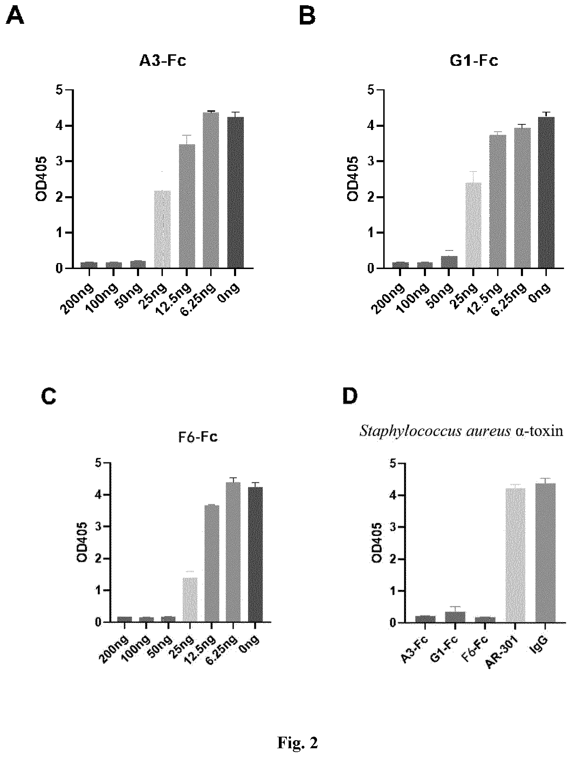

Above alpha toxin induced rabbit erythrocyte toxin model was used to detect neutralization of *Staphylococcus aureus* α- toxin by A3-Fc fusion proteins binding with antigen, G1-Fc fusion proteins binding with antigen and F6-Fc fusion proteins binding with antigen. The results of the neutralization effect are shown in Fig. 2, indicating that for the 18.75 ng recombinant alpha toxin, 50ng of A3-Fc fusion proteins binding with antigen (A), G1-Fc fusion proteins binding with antigen (B), and F6-Fc fusion proteins binding with antigen (C) had complete neutralization effect; the 50ng control antibody AR-301 could not completely neutralize 18.75ng recombinant *Staphylococcus aureus* alpha toxin (D), which indicates that the A3-Fc fusion proteins binding with antigen, G1-Fc fusion proteins binding with antigen and F6-Fc fusion proteins binding with antigen disclosed by the invention had significantly better neutralization effects on *Staphylococcus aureus* alpha toxin than that of the control antibody AR-301.

### Example 8. Replication of mouse sepsis model induced by Staphylococcus aureus alpha toxin and pharmacodynamic evaluation of the antigen binding protein

Different doses of antigen binding proteins were injected into C57BL/6 mice in advance, and 2.5 µg recombinant *Staphylococcus aureus* alpha toxin protein was injected into the tail vein after 30 minutes, using human IgG as a control. The survival time of mice was recorded.

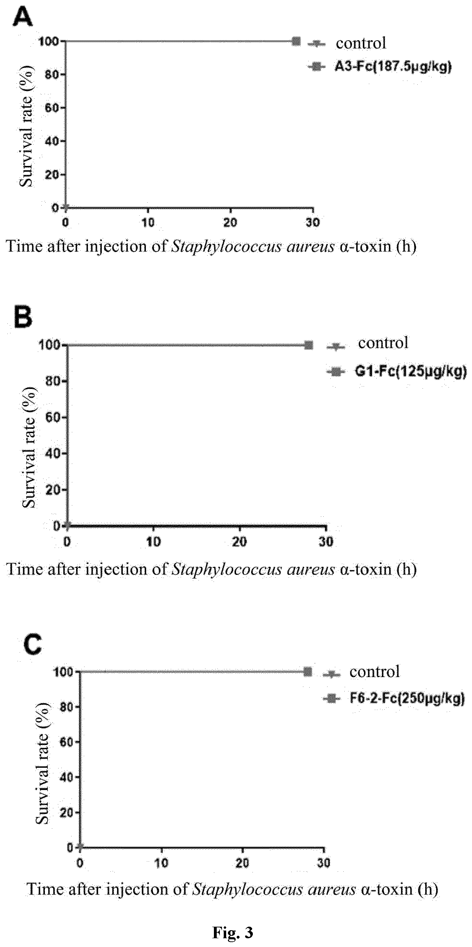

The mouse sepsis model induced by *Staphylococcus aureus* alpha toxin was used to detect the therapeutic effects of A3-Fc fusion proteins binding with antigen, G1-Fc fusion proteins binding with antigen, and F6-Fc fusion proteins binding with antigen, the results were shown in Fig. 3. The results indicated that the three antigen-binding proteins described herein: A3-Fc antigen-binding protein (187.5 µg/kg, A), G1-Fc fusion proteins binding with antigen (125 µg/kg, B), and F6-Fc fusion proteins binding with antigen (250 µg/kg, C) had a complete protective effect.

### Example 9. Replication of bacteremia model in mice infected with Staphylococcus aureus and evaluation of the therapeutic effect of the antigen binding protein

*Staphylococcus aureus* USA300 from -80°C refrigerator was activated in TSA medium for two generations, and inoculated to 2ml TSB for 12h. After washing 3 times with PBS, the tail vein of mice was infected with 6 × 10⁷ CFU. After 2 hours of infection, the antigen binding protein was injected with human IgG as a control, and the survival of mice was recorded.

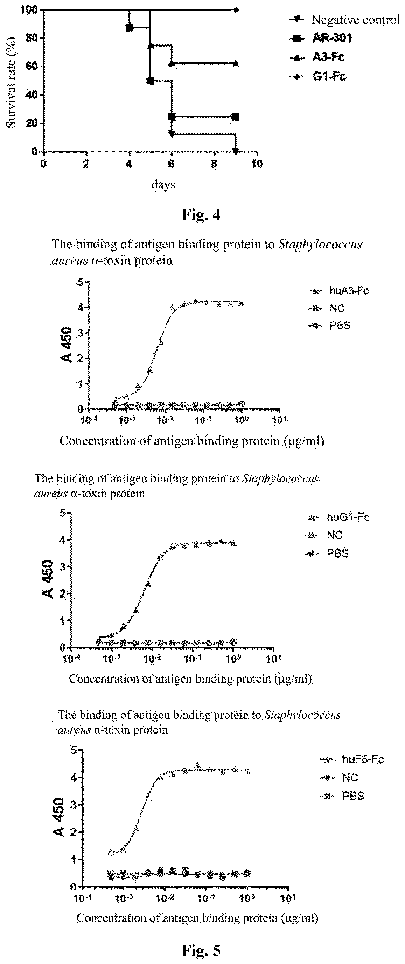

The results of evaluating the anti-infective pharmacodynamics of A3-Fc fusion proteins binding with antigen and G1-Fc fusion proteins binding with antigen using the mouse bacteremia model induced by *Staphylococcus aureus* are shown in Fig. 4. The results showed that A3-Fc fusion proteins binding with antigen and G1-Fc fusion proteins binding with antigen had significant protective effects on the mouse bacteremia model induced by *Staphylococcus aureus,* and the pharmacodynamics was better than the control antibody AR-301.

### Example 10. Humanization of antigen binding proteins of Staphylococcus aureus alpha toxin

By comparison through NCBI database (https://www.ncbi.nlm.nih.gov/igblast/), the human germline sequence closest to camel derived antibody was selected as the template to humanize A3, G1 and F6, and the post-translational modification sites of the antigen binding proteins were optimized.

Wherein, the humanized sequences of the obtained antigen binding proteins were as follows:
huA3 SEQ ID NO: 13 
QVQLVESGGGLVQPGGSLRLSCAASGFTFSRHGMTWVRQAPGKGLEYVGEINIPGDD
VDYDISVKGRFTISRDNAKNTLYLQMNSLRAEDTAVYYCARAGENMRLVTRGQGTLVTVS
S
huG1 SEQ ID NO: 25 
QVQLVESGGGLVQPGGSLRLSCAASGFTISSTGMTWVRQAPGKGLEWVGDINIPGTDT
DYDISVKGRFTISKDHAKNTVYLQMNSLRAEDTAVYYCAKQGANLRQGVQGQGTLVTVSS
 huF6 SEQ ID NO: 38

EVQLVESGGGLVQPGGSLRLSCAASGSTLDSYTVAWVRQAPGKGLEWVSCASRSGAS
TNYANSVKGRFTISRDNSKNTVYLQMNSLRAEDTAVYYCAAYRNFGNFCVMGRYEYDEW
GQGTLVTVSS

Wherein, the underlined part represents the CDR regions generated using the Chothia numbering scheme.

The variable region coding sequences of the above antigen binding proteins huA3, huG1, and huF6 were fused with the Fc fragment of human immunoglobulin γ (IgG1), respectively, and subcloned into expression vector PCDNA3.4. Expi293 cells were transfected with PEI for recombinant expression. After 7 days, the cell supernatant was collected and purified using ProteinA magnetic beads. Antigen binding proteins with a purity of over 95% was collected, and huA3-Fc fusion proteins binding with antigen, huG1-Fc fusion proteins binding with antigen, and huF6-Fc fusion proteins binding with antigen were obtained. The humanized sequences of the above antigen binding proteins are as follows:
huA3-Fc SEQ ID NO: 68 
QVQLVESGGGLVQPGGSLRLSCAASGFTFSRHGMTWVRQAPGKGLEYVGEINIPGDD
VDYDISVKGRFTISRDNAKNTLYLQMNSLRAEDTAVYYCARAGENMRLVTRGQGTLVTVS
SEPKSSDKTHTCPPCPAPELLGGPSVFLFPPKPKDTLYITREPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGK
huG1-Fc SEQ ID NO: 69 
QVQLVESGGGLVQPGGSLRLSCAASGFTISSTGMTWVRQAPGKGLEWVGDINIPGTDT
DYDISVKGRFTISKDHAKNTVYLQMNSLRAEDTAVYYCAKQGANLRQGVQGQGTLVTVSS
EPKSSDKTHTCPPCPAPELLGGPSVFLFPPKPKDTLYITREPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGK
 huF6-Fc SEQ ID NO: 70

EVQLVESGGGLVQPGGSLRLSCAASGSTLDSYTVAWVRQAPGKGLEWVSCASRSGAS
TNYANSVKGRFTISRDNSKNTVYLQMNSLRAEDTAVYYCAAYRNFGNFCVMGRYEYDEW
GQGTLVTVSSEPKSSDKTHTCPPCPAPELLGGPSVFLFPPKPKDTLYITREPEVTCVVVDVSH
EDPEVKFNWYVDGVEVHNAKTKPREEQYNSTYRVVSVLTVLHQDWLNGKEYKCKVSNK
ALPAPIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPGK

The binding activity of above antigen binding protein with *Staphylococcus aureus* alpha toxin was detected according to the method of Example 6. The experimental results are shown in Fig. 5, indicating that above three antigen binding proteins were associated with *Staphylococcus aureus* alpha toxin with a strong affinity, with an EC₅₀ value of 0.005970 µg/ml for the huA3-Fc fusion proteins binding with antigen, an EC₅₀ value of 0.006353 µg/ml for huG1-Fc fusion proteins binding with antigen, and an EC₅₀ value of 0.002721 µg/ml for huF6-Fc fusion proteins binding with antigen.

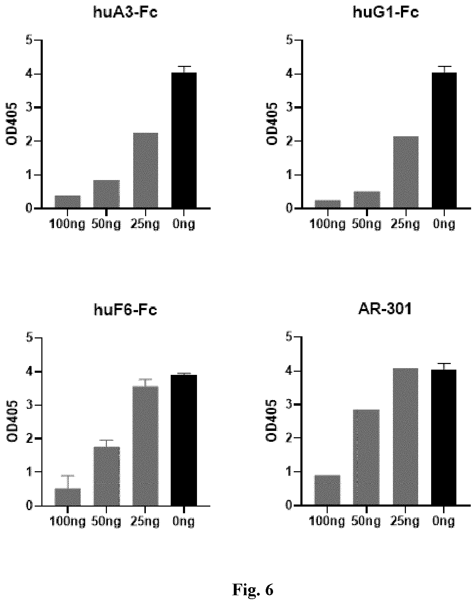

The neutralization effects of above antigen binding proteins on the hemolytic activity of *Staphylococcus aureus* alpha toxin were evaluated in the model of rabbit erythrocytes toxin induced by alpha toxin in Example 7. The experimental results are shown in Fig. 6, indicating that huA3-Fc, huG1-Fc, and huF6-Fc have significantly better effects on the neutralization effect of *Staphylococcus aureus* alpha toxin than that of the control antibody AR-301.

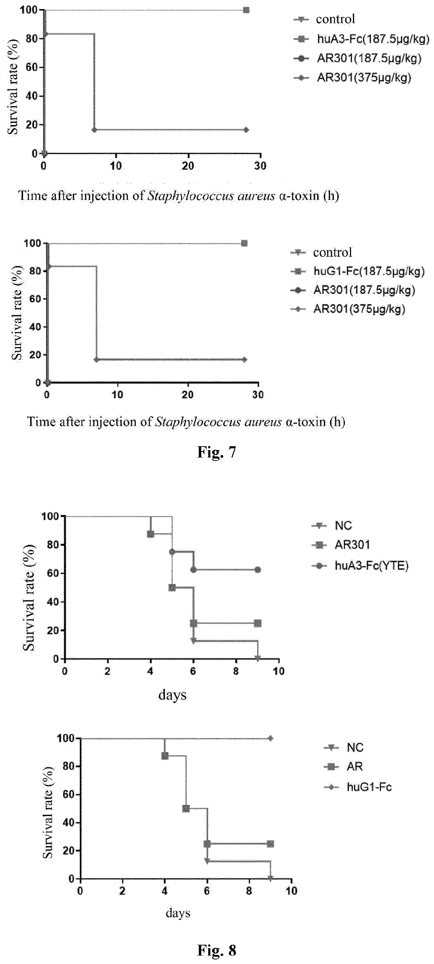

The efficacy of antigen binding proteins was evaluated using a mouse sepsis model induced by *Staphylococcus aureus* alpha toxin. Different doses of antigen binding proteins were injected into C57BL/6 mice in advance, and 2.5µg recombinant *Staphylococcus aureus* alpha toxin protein was injected into the tail vein after 30 minutes, using human IgG as control. The survival time of mice was recorded. The results of the therapeutic effects of huA3-Fc fusion proteins binding with antigen and huG1-Fc fusion proteins binding with antigen on a mouse sepsis model induced by *Staphylococcus aureus* alpha toxin were shown in Fig. 7. The results indicated that the protective effects of the three antigen-binding proteins described herein: A3-Fc antigen-binding protein and G1-Fc antigen-binding protein are superior to that of AR301.

The therapeutic effect of the antigen binding proteins was evaluated using the model of bacteremia caused by *Staphylococcus aureus* infection in mice. *Staphylococcus aureus* USA300 from -80°C refrigerator was activated in TSA medium for two generations, and inoculated to 2ml TSB for 12h. After washing 3 times with PBS, 6 × 10⁷ CFU was infected in the tail vein of mice. 2 hours after infection, the antigen binding protein was injected with human IgG as a control, and the survival of mice was recorded. The results of the anti-infective pharmacodynamics of huA3-Fc fusion proteins binding with antigen and huG1-Fc fusion proteins binding with antigen were evaluated using the mouse bacteremia model caused by *Staphylococcus aureus,* and the results were shown in Fig. 8. The results showed that huA3-Fc fusion proteins binding with antigen and huG1-Fc fusion proteins binding with antigen had significant protective effects on the mouse bacteremia model caused by *Staphylococcus aureus,* and its pharmacodynamics was better than the control antibody AR-301.

### Example 11. Preparation of tetravalent Fc fusion protein binding with antigen of Staphylococcus aureus alpha toxin

Two anti*-Staphylococcus aureus* alpha toxin antigen sequences (e.g., two huA3 molecules or two huG1 molecules or one huA3 molecule and one huG1 molecule) were connected in series with the nucleic acid fragment encoding the human immunoglobulin G1 (IgG1) Fc, and subcloned into pcDNA3.4 plasmid, and expressed and purified by the method in Example 5. The recombinantly expressed molecule contains 4 binding domains associated with *Staphylococcus aureus* alpha toxin, which were called tetravalent Fc-fusion proteins binding with antigen of *Staphylococcus aureus* alpha toxin. The amino acid sequence of the huA3-huA3-Fc fusion proteins binding with antigen is shown in SEQ ID NO: 65, the amino acid sequence of the huG1-huG1-Fc fusion protein is shown in SEQ ID NO: 66, and the amino acid sequence of the huA3-huG1-Fc fusion proteins binding with antigen is shown in SEQ ID NO: 67.

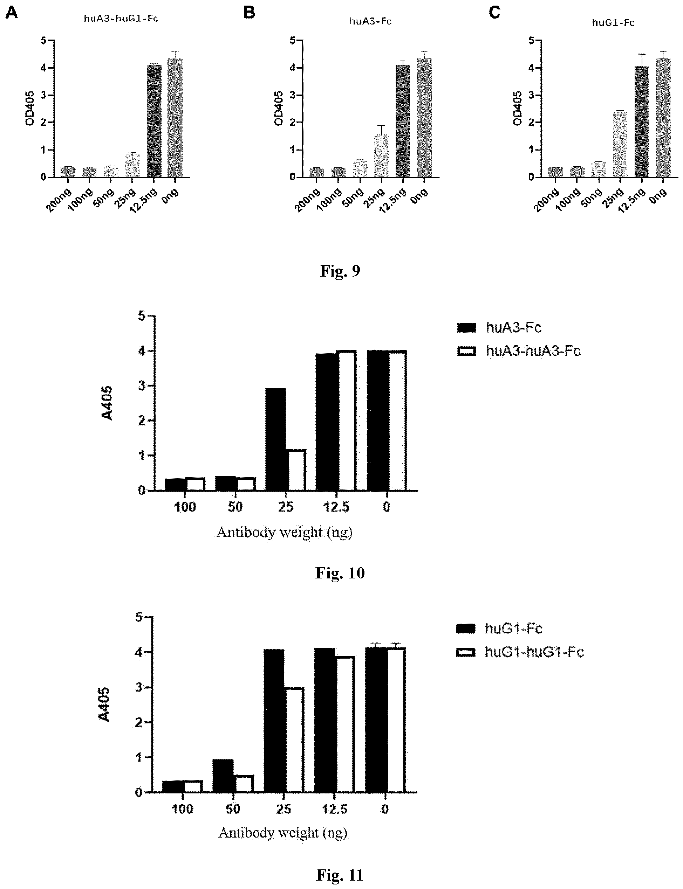

The neutralization effects of tetravalent huA3-huG1-Fc fusion protein binding with antigen , huA3-Fc fusion protein binding with antigen and huG1-Fc fusion protein binding with antigen on *Staphylococcus aureus* alpha toxin were detected using the model of rabbit erythrocyte toxin induced by *Staphylococcus aureus* in Example 7, and the results were shown in Fig. 9. The results showed that for 18.75ng recombinant *Staphylococcus aureus* alpha toxin, the neutralization effect of tetravalent huA3-huG1-Fc fusion protein was superior to that of divalent huA3-Fc fusion protein binding with antigen and huG1-Fc fusion protein binding with antigen at the same dose.

The neutralization effects of tetravalent huA3-huA3-Fc fusion protein binding with antigen , huA3-Fc fusion protein binding with antigen on *Staphylococcus aureus* alpha toxin were detected using the model of rabbit erythrocyte toxin induced by *Staphylococcus aureus* in Example 7, and the results were shown in Fig. 10. The results showed that for 18.75ng recombinant *Staphylococcus aureus* alpha toxin, the neutralization effect of tetravalent huA3-huA3-Fc fusion protein was superior to that of divalent huA3-Fc fusion protein binding with antigen at the same dose.

The neutralization effects of tetravalent huG1-huG1-Fc fusion proteins binding with antigen, huG1-Fc fusion proteins binding with antigen on *Staphylococcus aureus* alpha toxin were detected using the model of rabbit erythrocyte toxin induced by *Staphylococcus aureus* in Example 7, and the results were shown in Fig. 11. The results showed that for 18.75ng recombinant *Staphylococcus aureus* alpha toxin, the neutralization effect of tetravalent huG1-huG1-Fc fusion protein was superior to that of divalent huG1-Fc fusion proteins binding with antigen at the same dose.

## Claims

1. An isolated antigen binding protein comprising a HCDR3, wherein the HCDR3 comprises an amino acid sequence shown in SEQ ID NO: 1, SEQ ID NO: 14, or SEQ ID NO: 26.

2. The isolated antigen binding protein according to claim 1, which comprises a HCDR2, the HCDR2 comprises an amino acid sequence shown in SEQ ID NO: 52 or SEQ ID NO: 27.

3. The isolated antigen binding protein according to claim 2, wherein the HCDR2 comprises an amino acid sequence shown in SEQ ID NO: 2 or SEQ ID NO: 15.

4. The isolated antigen binding protein according to any one of claims 1-3, which comprises a HCDR1, the HCDR1 comprises an amino acid sequence shown in SEQ ID NO: 53 or SEQ ID NO: 28.

5. The isolated antigen binding protein according to claim 4, wherein the HCDR1 comprises an amino acid sequence shown in SEQ ID NO: 3 or SEQ ID NO: 16.

6. The isolated antigen binding protein according to any one of claims 1-5, comprising HCDR1, HCDR2, and HCDR3, the HCDR1 comprises an amino acid sequence shown in SEQ ID NO: 53 or SEQ ID NO: 28, the HCDR2 comprises an amino acid sequence shown in SEQ ID NO: 52 or SEQ ID NO: 27, and the HCDR3 comprises an amino acid sequence shown in SEQ ID NO: 1, SEQ ID NO: 14, or SEQ ID NO: 26.

7. The isolated antigen binding protein according to any one of claims 1-6, comprising HCDR1, HCDR2, and HCDR3, the HCDR1 comprises an amino acid sequence shown in any one of SEQ ID NO: 3, SEQ ID NO: 16, and SEQ ID NO: 28, the HCDR2 comprises an amino acid sequence shown in any one of SEQ ID NO: 2, SEQ ID NO: 15, and SEQ ID NO: 27, and the HCDR3 comprises an amino acid sequence shown in any one of SEQ ID NO: 1, SEQ ID NO: 14, and SEQ ID NO: 26.

8. The isolated antigen binding protein according to any one of claims 1-7, comprising HCDR1, HCDR2, and HCDR3 selected from any of the following groups:
1) the HCDR1 comprises an amino acid sequence shown in SEQ ID NO: 3, the HCDR2 comprises an amino acid sequence shown in SEQ ID NO: 2, and the HCDR3 comprises an amino acid sequence shown in SEQ ID NO: 1;
2) the HCDR1 comprises an amino acid sequence shown in SEQ ID NO: 16, the HCDR2 comprises an amino acid sequence shown in SEQ ID NO: 15, and the HCDR3 comprises an amino acid sequence shown in SEQ ID NO: 14; and
3) the HCDR1 comprises an amino acid sequence shown in SEQ ID NO: 28, the HCDR2 comprises an amino acid sequence shown in SEQ ID NO: 27, and the HCDR3 comprises an amino acid sequence shown in SEQ ID NO: 26.

9. The isolated antigen binding protein according to any one of claims 4-8, comprising a H-FR1, the C-terminus of the H-FR1 is directly or indirectly linked to the N-terminus of the HCDR1, and the H-FR1 comprises an amino acid sequence shown in SEQ ID NO: 54.

10. The isolated antigen binding protein according to claim 9, wherein the H-FR1 comprises an amino acid sequence shown in any of SEQ ID NO: 4, SEQ ID NO: 8, SEQ ID NO: 17, SEQ ID NO: 29, and SEQ ID NO: 33.

11. The isolated antigen binding protein according to any one of claims 4-10, which comprises a H-FR2 located between HCDR1 and HCDR2, and the H-FR2 comprises an amino acid sequence shown in SEQ ID NO: 55 or SEQ ID NO: 56.

12. The isolated antigen binding protein according to claim 11, wherein the H-FR2 comprises an amino acid sequence shown in any of SEQ ID NO: 5, SEQ ID NO: 9, SEQ ID NO: 18, SEQ ID NO: 21, SEQ ID NO: 30, and SEQ ID NO: 34.

13. The isolated antigen binding protein according to any one of claims 2-12, which comprises a H-FR3 located between HCDR2 and HCDR3, and the H-FR3 comprises an amino acid sequence shown in SEQ ID NO: 57 or SEQ ID NO: 58.

14. The isolated antigen binding protein according to claim 13, wherein the H-FR3 comprises an amino acid sequence shown in any of SEQ ID NO: 6, SEQ ID NO: 10, SEQ ID NO: 19, SEQ ID NO: 22, SEQ ID NO: 31, and SEQ ID NO: 35.

15. The isolated antigen binding protein according to any one of claims 1-14, which comprises a H-FR4, the N-terminus of the H-FR4 is linked to the C-terminus of HCDR3, and the the H-FR4 comprises an amino acid sequence shown in SEQ ID NO: 59.

16. The isolated antigen binding protein according to claim 15, wherein the H-FR4 comprises an amino acid sequence shown in any of SEQ ID NO: 7, SEQ ID NO: 11, SEQ ID NO: 20, SEQ ID NO: 23, SEQ ID NO: 32, and SEQ ID NO: 36.

17. The isolated antigen binding protein according to any one of claims 1-16, comprising H-FR1, H-FR2, H-FR3, and H-FR4, the H-FR1 comprises an amino acid sequence shown in SEQ ID NO: 54, the H-FR2 comprises an amino acid sequence shown in SEQ ID NO: 55 or SEQ ID NO: 56, the H-FR3 comprises an amino acid sequence shown in SEQ ID NO: 57 or SEQ ID NO: 58, and the H-FR4 comprises an amino acid sequence shown in SEQ ID NO: 59.

18. The isolated antigen binding protein according to any one of claims 1-17, comprising H-FR1, H-FR2, H-FR3, and H-FR4, the H-FR1 comprises an amino acid sequence shown in any one of SEQ ID NO: 4, SEQ ID NO: 8, SEQ ID NO: 17, SEQ ID NO: 29, and SEQ ID NO: 33, the H-FR2 comprises an amino acid sequence shown in any one of SEQ ID NO: 5, SEQ ID NO: 9, SEQ ID NO: 18, SEQ ID NO: 21, SEQ ID NO: 30, and SEQ ID NO: 34, the H-FR3 comprises an amino acid sequence shown in any of SEQ ID NO: 6, SEQ ID NO: 10, SEQ ID NO: 19, SEQ ID NO: 22, SEQ ID NO: 31, and SEQ ID NO: 35, and the H-FR4 comprises an amino acid sequence shown in any of SEQ ID NO: 7, SEQ ID NO: 11, SEQ ID NO: 20, SEQ ID NO: 23, SEQ ID NO: 32, and SEQ ID NO: 36.

19. The isolated antigen binding protein according to any one of claims 1-18, which comprises H-FR1, H-FR2, H-FR3, and H-FR4 selected from any of the following groups:
1) the H-FR1 comprises an amino acid sequence shown in SEQ ID NO: 4, the H-FR2 comprises an amino acid sequence shown in SEQ ID NO: 5, the H-FR3 comprises an amino acid sequence shown in SEQ ID NO: 6, and the H-FR4 comprises an amino acid sequence shown in SEQ ID NO: 7;
2) the H-FR1 comprises an amino acid sequence shown in SEQ ID NO: 8, the H-FR2 comprises an amino acid sequence shown in SEQ ID NO: 9, the H-FR3 comprises an amino acid sequence shown in SEQ ID NO: 10, and the H-FR4 comprises an amino acid sequence shown in SEQ ID NO: 11;
3) the H-FR1 comprises an amino acid sequence shown in SEQ ID NO: 17, the H-FR2 comprises an amino acid sequence shown in SEQ ID NO: 18, the H-FR3 comprises an amino acid sequence shown in SEQ ID NO: 19, and the H-FR4 comprises an amino acid sequence shown in SEQ ID NO: 20;
4) the H-FR1 comprises an amino acid sequence shown in SEQ ID NO: 8, the H-FR2 comprises an amino acid sequence shown in SEQ ID NO: 21, the H-FR3 comprises an amino acid sequence shown in SEQ ID NO: 22, and the H-FR4 comprises an amino acid sequence shown in SEQ ID NO: 23;
5) the H-FR1 comprises an amino acid sequence shown in SEQ ID NO: 29, the H-FR2 comprises an amino acid sequence shown in SEQ ID NO: 30, the H-FR3 comprises an amino acid sequence shown in SEQ ID NO: 31, and the H-FR4 comprises an amino acid sequence shown in SEQ ID NO: 32; and
6) the H-FR1 comprises an amino acid sequence shown in SEQ ID NO: 33, the H-FR2 comprises an amino acid sequence shown in SEQ ID NO: 30, the H-FR3 comprises an amino acid sequence shown in SEQ ID NO: 35, and the H-FR4 comprises an amino acid sequence shown in SEQ ID NO: 36.

20. The isolated antigen binding protein according to any one of claims 1-19, comprising a VH, the VH comprises an amino acid sequence shown in SEQ ID NO: 60 or SEQ ID NO: 61.

21. The isolated antigen binding protein according to claim 20, wherein the VH comprises an amino acid sequence shown in any of SEQ ID NO: 12, SEQ ID NO: 13, SEQ ID NO: 24, SEQ ID NO: 25, SEQ ID NO: 37, and SEQ ID NO: 38.

22. The isolated antigen binding protein according to any one of claims 1-21, comprising VHH.

23. The isolated antigen binding protein according to claim 22, wherein the VHH comprises an amino acid sequence shown in SEQ ID NO: 60 or SEQ ID NO: 61.

24. The isolated antigen binding protein according to any one of claims 22-23, wherein the VHH comprises an amino acid sequence shown in any one of SEQ ID NO: 12, SEQ ID NO: 13, SEQ ID NO: 24, SEQ ID NO: 25, SEQ ID NO: 37, and SEQ ID NO: 38.

25. The isolated antigen binding protein according to any one of claims 1-24, comprising an heavy chain constant region of an antibody.

26. The isolated antigen binding protein according to claim 25, wherein the heavy chain constant region of an antibody is derived from a human IgG constant region.

27. The isolated antigen binding protein according to any one of claims 25-26, wherein the heavy chain constant region of an antibody is derived from a constant region of a human IgG1.

28. The isolated antigen binding protein according to any one of claims 25-27, wherein the heavy chain constant region of an antibody comprises an amino acid sequence as shown in SEQ ID NO: 39.

29. The isolated antigen binding protein according to any one of claims 1-28, which comprises an amino acid sequence shown in any one of SEQ ID NO: 62, SEQ ID NO: 63, SEQ ID NO: 64, SEQ ID NO: 68, SEQ ID NO: 69, and SEQ ID NO: 70.

30. The isolated antigen binding protein according to any one of claims 1-29, comprising an antibody or an antigen binding fragment thereof.

31. The isolated antigen binding protein according to claim 30, wherein the antigen binding fragment includes Fab, Fab', Fv fragment, Bs-Fv, F(ab')₂, F(ab)₂, scFv, di-scFv, and/or dAb.

32. The isolated antigen binding protein according to any one of claims 30-31, wherein the antibody is selected from one or more of the following groups: monoclonal antibodies, chimeric antibodies, humanized antibodies, and fully human antibodies.

33. The isolated antigen binding protein according to any one of claims 1-32, which can specifically bind to *Staphylococcus aureus* α-toxin.

34. The isolated antigen binding protein according to any one of claims 1-33, which can neutralize the biological activity of *Staphylococcus aureus* α-toxin.

35. The isolated antigen binding protein according to any one of claims 1-34, which can prevent and/or treat a disease and/or a condition and a complication thereof.

36. The isolated antigen binding protein according to claim 35, wherein the disease and/or condition and complication thereofcomplication thereof are caused or mediated by *Staphylococcus aureus.*

37. The isolated antigen binding protein according to any one of claims 35-36, wherein the disease and/or condition and complication thereof include bacteremia and/or sepsis.

38. A polypeptide molecule, comprising the isolated antigen binding protein according to any one of claims 1-37.

39. The polypeptide molecule according to claim 38, comprising a fusion protein.

40. The polypeptide molecule according to any one of claims 38-39, comprising two or more isolated antigen binding proteins according to any one of claims 1-37 or a fusion protein of two or more isolated antigen binding proteins according to any one of claims 1-37 and an antibody heavy chain constant region as described in any one of claims 25-28.

41. The polypeptide molecule according to any one of claims 38-40, comprising an amino acid sequence shown in any one of SEQ ID NO: 65, SEQ ID NO: 66, and SEQ ID NO: 67.

42. An immunoconjugate, comprising the isolated antigen binding protein according to any one of claims 1-37.

43. A nucleic acid molecule encoding the isolated antigen binding protein according to any one of claims 1-37 or thje polypeptide molecule according to any one of claims 38-41.

44. A vector comprising the nucleic acid molecule according to claim 43.

45. A cell comprising the nucleic acid molecule according to claim 43 or the vector according to claim 44.

46. A pharmaceutical composition comprising the isolated antigen binding protein according to any one of claims 1-37, the polypeptide molecule according to any one of claims 38-41, the immunoconjugate according to claim 42, the nucleic acid molecule according to claim 43, the vector according to claim 44, and/or the cell according to claim 45, and optionally a pharmaceutically acceptable vehicle.

47. A method for preparing an isolated antigen binding protein according to any one of claims 1-37, wherein the method comprises culturing the cells according to claim 45 under conditions that enable the expression of the antigen binding protein.

48. Use of the isolated antigen binding protein according to any one of claims 1-37, the peptide molecule according to any one of claims 38-41, the immunoconjugate according to claim 42, the nucleic acid molecule according to claim 43, the vector according to claim 44, the cell according to claim 45, and/or the pharmaceutical composition according to claim 46 in the manufacture of a medicament for preventing and/or treating a disease and/or condition and complication thereof.

49. The use according to claim 48, wherein the disease and/or condition and complication thereof is caused or mediated by *Staphylococcus aureus.*

50. The use according to any one of claims 48-49, wherein the disease and/or condition and complication thereof includes sepsis and/or bacteremia.

51. The isolated antigen binding protein according to any one of claims 1-37, the polypeptide molecule according to any one of claims 38-41, the immunoconjugate according to claim 42, the nucleic acid molecule according to claim 43, the vector according to claim 44, the cell according to claim 45, and/or the pharmaceutical combination according to claim 46, which are used alone or in combination with other drugs.

52. A method for detecting *Staphylococcus aureus* α-toxin in a sample, comprising administrating the isolated antigen binding protein according to any one of claims 1-37, the polypeptide molecule according to any one of claims 38-41, the immunoconjugate according to claim 42, the nucleic acid molecule according to claim 43, the vector according to claim 44, the cell according to claim 45, and/or the pharmaceutical combination according to claim 46.

53. A reagent or kit for detecting *Staphylococcus aureus* α-toxin in a sample, comprising the isolated antigen binding protein according to any one of claims 1-37, the polypeptide molecule according to any one of claims 38-41, the immunoconjugate according to claim 42, the nucleic acid molecule according to claim 43, the vector according to claim 44, the cell according to claim 45, and/or the pharmaceutical combination according to claim 46.

54. Use of the isolated antigen binding protein according to any one of claims 1-37, the polypeptide molecule according to any one of claims 38-41, the immunoconjugate according to claim 42, the nucleic acid molecule according to claim 43, the vector according to claim 44, the cell according to claim 45, and/or the pharmaceutical combination according to claim 46 in the manufacture of a kit for detecting the presence and/or content of *Staphylococcus aureus* α-toxin in a sample.
